# EUROPÄISCHE PATENTSCHRIFT

(11) **EP 2 875 027 B1**
(45) Veröffentlichungstag und Bekanntmachung des Hinweises auf die Patenterteilung: **02.05.2018**
(21) Anmeldenummer: 13741712.7
(22) Anmeldetag: 22.07.2013
(51) Int. Cl.: C07D 493/04, C07B 43/04, B01J 23/46

(54) **VERFAHREN ZUR HOMOGEN-KATALYSIERTEN AMINIERUNG VON ALKOHOLEN MIT AMMONIAK IN GEGENWART EINES KOMPLEXKATALYSATORS OHNE ANIONISCHE LIGANDEN**
PROCESS FOR HOMOGENEOUSLY CATALYSED AMINATION OF ALCOHOLS WITH AMMONIA IN THE PRESENCE OF A COMPLEX CATALYST WITHOUT ANIONIC LIGANDS
PROCÉDÉ D'AMINATION D'ALCOOLS À L'AMMONIAQUE SOUS CATALYSE HOMOGÈNE EN PRÉSENCE D'UN CATALYSEUR COMPLEXE SANS LIGANDS ANIONIQUES

(30) Priorität: 23.07.2012 EP 12177529
(43) Veröffentlichungstag der Anmeldung: 27.05.2015
(73) Patentinhaber: BASF SE, 67056 Ludwigshafen am Rhein (DE)
(72) Erfinder: SCHELWIES, Mathias, 69115 Heidelberg (DE); DA SILVA, Marion, 68165 Mannheim (DE); SCHAUB, Thomas, 67433 Neustadt (DE); MELDER, Johann-Peter, 67459 Böhl-Iggelheim (DE); PACIELLO, Rocco, 67098 Bad Dürkheim (DE); MERGER, Martin, 67227 Frankenthal (DE)
(86) Internationale Anmeldenummer: PCT/EP2013/065402
(87) Internationale Veröffentlichungsnummer: WO 2014/016241

(56) Entgegenhaltungen:
- WO-A1-2011/151268
- IMM S ET AL: "Improved Ruthenium-Catalyzed Amination of Alcohols with Ammonia: Synthesis of Diamines and Amino Esters", ANGEWANDTE CHEMIE. INTERNATIONAL EDITION, WILEY VCH VERLAG, WEINHEIM, Bd. 50, Nr. 33, 8. August 2011 (2011-08-08), Seiten 7599-7603, XP002664616, ISSN: 1433-7851, DOI: 10.1002/ANIE.201103199 [gefunden am 2011-07-05] in der Anmeldung erwähnt

## Beschreibung

Die vorliegende Erfindung betrifft ein Verfahren zur Herstellung von primären Aminen durch Alkoholaminierung von Alkoholen mit Ammoniak unter Wasserabspaltung in Gegenwart mindestens eines Komplexkatalysators der allgemeinen Formel (I), der Ruthenium sowie mindestens einen mindestens bidentalen Donorliganden enthält.

Primäre Amine sind Verbindungen, die mindestens eine primäre Aminogruppe (-NH₂) aufweisen. Primäre Amine sind wertvolle Produkte mit einer Vielzahl unterschiedlicher Verwendungen, beispielsweise als Lösungsmittel oder Stabilisatoren, zur Synthese von Chelat-Bildnern, als Edukte zur Herstellung von Kunstharzen, Inhibitoren, grenzflächenaktiven Substanzen, als Zwischenprodukte bei der Herstellung von Kraftstoffadditiven, Tensiden, Arznei- und Pflanzenschutzmitteln, Härtern für Epoxyharze, Katalysatoren für Polyurethane, als Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

Primäre Amine werden gegenwärtig durch die heterogen-katalysierte Alkoholaminierung von Alkoholen mit Ammoniak hergestellt. Die WO 2008/006752 A1 beschreibt ein Verfahren zur Herstellung von Aminen durch Umsetzung von primären oder sekundären Alkoholen mit Ammoniak in Gegenwart eines heterogenen Katalysators, der Zirkoniumdioxid und Nickel enthält. Die Reaktion wird bei Temperaturen im Bereich von 150 bis 210 °C und Ammoniakdrücken im Bereich von 30 bis 200 bar durchgeführt.

Die homogen-katalysierte Aminierung von Monoalkoholen mit primären und sekundären Aminen ist seit den 1970er Jahren bekannt und wird beispielsweise in der US 3,708,539 beschrieben. Hierbei kommen Ruthenium- oder Osmiumkatalysatoren zum Einsatz, die Triphenylphosphin und anionische Liganden wie Hydrid, Halogenid oder Nitrat enthalten. Mit den in der US 3,708,539 beschriebenen Katalysatorsystemen ist nur die Aminierung von Monoalkoholen mit primären und sekundären Aminen möglich. Die Umsetzung von Alkoholen mit Ammoniak, welches die wirtschaftlich attraktivste Aminierungsreaktion darstellt, wird in dieser Arbeit nicht beschrieben.

WO 2010/018570 und C. Gunanathan, D. Milstein, Angew. Chem. Int. Ed. 2008, 47, 8661-8664 beschreiben die Aminierung von primären Monoalkoholen mit Ammoniak zu primären Monoaminen in Gegenwart eines Ruthenium-Komplexkatalysators. Das im Komplexkatalysator enthaltene Ruthenium ist dabei an einen Acridinyl-basierten Liganden komplexiert, der zusätzlich Isopropyl-substituierte Phosphingruppen aufweist. Das Ruthenium im Komplexkatalysator trägt dabei zwei anionische Liganden sowie Kohlenmonoxid und entspricht der folgenden Formel:

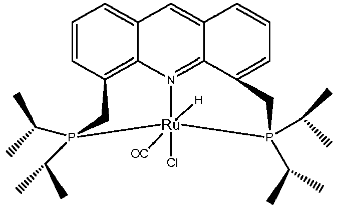

Der Komplexkatalysator wird aus [RuHCl(PPh₃)₃(CO)] in Toluol unter Zugabe des entsprechenden Liganden (4,5-Bis-(di-iso-propylphosphinomethyl)acridin) und nachfolgender Entfernung des Lösungsmittels hergestellt.

An das Ruthenium des Komplexkatalysators sind ein Chloridanion und ein Hydridanion gebunden. Dies wird auch als Rutheniummonohydrid bezeichnet. Die Umsetzung von sekundären Alkoholen sowie Di-, Tri- oder Polyolen zu primären Mono-, Di-, Tri- oder Polyaminen ist in WO 2010/018570 nicht beschrieben.

Nachteilig an dem vorstehend beschriebenen Komplexkatalysator ist, dass dieser zunächst in einem der Alkoholaminierung vorgelagerten zweistufigen Verfahren bereitgestellt werden muss.

S. Imm, S. Bähn, L. Neubert, H. Neumann, M. Beller, Angew. Chem. 2010, 122, 8303-8306 und D. Pingen, C. Müller, D. Vogt, Angew. Chem. 2010, 122, 8307-8310 beschreiben die mit Rutheniumkatalysatoren homogen-katalysierte Aminierung von sekundären Alkoholen wie Cyclohexanol mit Ammoniak. Als Phosphordonorliganden werden hier monodentate Phosphinliganden eingesetzt.
Nachteilig an diesen Verfahren ist, dass die notwendigen Katalysatorladungen mit 6mol% bezogen auf das Substrat hoch sind.

DE 10 2011 004 465 und S. lmm, S. Bähn, M. Zang, L. Neubert, H. Neumann, F. Klasovsky, J. Pfeffer, T. Haas und M. Beller, Angew. Chem. 2011, 123, 7741-7745 beschreiben ein Verfahren zur homogenkatalysierten Aminierung von sekundären Alkoholen mit Ammoniak. Gute Ergebnisse werden dabei mit einem Komplexkatalysator erzielt, der aus Xantphos (4,5-Bis-(diphenylphosphino)-9,9-dimethylxanthen) und [Ru(CO)ClH(PPh₃)₃] zugänglich ist. Gute Ergebnisse werden ebenfalls mit dem Komplexkatalysator Carbonylchlorohydrido-[4,5-(di-iso-propylphosphinomethyl-acridino)ruthenium (II)] erzielt. Dieser Komplexkatalysator entspricht der vorstehend zu WO 2010/018570 gezeigten Formel.

Auch die in der DE 10 2011 004 465 beschriebenen Komplexkatalysatoren weisen anionische Liganden auf, die an das Ruthenium komplexiert sind. Die in DE 10 2011 004 465 beschriebenen Komplexkatalysatoren weisen ebenfalls eine Ruthenium-Hydrid-Substruktur auf.

Obwohl im Stand der Technik die homogen-katalysierte Umsetzung von primären und sekundären Alkoholen zu primären Aminen beschrieben ist, besteht dennoch ein großer Bedarf an alternativen Verfahren, die sich bezüglich Aktivität und Selektivität von den bereits bekannten Katalysatorsystemen abheben und vorteilhaft in situ unter den Reaktionsbedingungen der Alkoholaminierung herstellbar sind.

Aufgabe der vorliegenden Erfindung ist es, ein Verfahren zur Herstellung von primären Aminen durch homogen-katalysierte Alkoholaminierung von Alkoholen, insbesondere sekundären Alkoholen mit Ammoniak unter Wasserabspaltung bereitzustellen. Das Verfahren soll die primären Amine in guten Ausbeuten und Selektivitäten liefern, und die Bildung von unerwünschten Nebenprodukten wie sekundären oder tertiären Aminen soll weitestgehend vermieden werden. Darüber hinaus soll das Verfahren auch den Einsatz eines einfacher zugänglichen Komplexkatalysators ermöglichen, der idealerweise direkt in situ unter den Reaktionsbedingungen der Alkoholaminierung zugänglich ist.

Gelöst wird die Aufgabe durch ein Verfahren zur Herstellung von primären Aminen durch Alkoholaminierung von Alkoholen mit Ammoniak unter Wasserabspaltung, wobei die Alkoholaminierung homogen-katalysiert in Gegenwart mindestens eines Komplexkatalysators durchgeführt wird, der Ruthenium sowie mindestens einen Donorliganden jedoch keinen anionischen Liganden enthält, wobei der Komplexkatalysator ein Katalysator der allgemeinen Formel (I) ist: 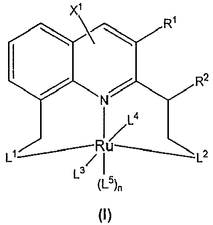 in der
- L¹ und L²: sind unabhängig voneinander PR^{a}R^{b} oder NR^{a}R^{b};
- L³, L⁴ und L⁵: sind unabhängig voneinander einzähnige Zweielektronendonoren ausgewählt sind aus der Gruppe CO und PR^{a}R^{b}R^{c};
- n: ist eine ganze Zahl 0 oder 1;
- R¹ und R²: sind beide Wasserstoffe oder bilden gemeinsam mit den Kohlenstoffatomen, an die sie gebunden sind, einen Phenylring, der zusammen mit der Chinolinyleinheit der Formel I eine Acridinyleinheit bildet;
- R, R^{a}, R^{b} und R^{c}: sind unabhängig voneinander unsubstituiertes oder zumindest monosubstituiertes C₁-C₁₀-Alkyl, C₅-C₁₀-Cycloalkyl, C₅-C₁₀-Heterocyclyl, C₅-C₁₀-Aryl oder C₅-C₁₀-Heteroaryl,
wobei die Substituenten ausgewählt sind aus der Gruppe bestehend aus F, Cl, Br, OH, CN, NH₂ und C₁-C₁₀-Alkyl;
- X¹: stellt ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit dar,
- wobei X¹: unabhängig voneinander ausgewählt ist aus der Gruppe bestehend aus Wasserstoff, F, Cl, Br, I, OH, NH₂, NO₂, NC(O)R, C(O)NR₂, OC(O)R, C(O)OR, CN und unsubstituiertem oder zumindest monosubstituiertem C₁-C₁₀-Alkoxy, C₁-C₁₀-Alkyl, C₅-C₁₀-Cycloalkyl, C₅-C₁₀-Heterocyclyl, C₅-C₁₀-Aryl und C₅-C₁₀-Heteroaryl,
wobei die Substituenten ausgewählt sind aus der Gruppe bestehend aus F, Cl, Br, OH, CN, NH₂ und C₁-C₁₀-Alkyl.

Überraschend wurde festgestellt, dass mit den im erfindungsgemäßen Verfahren eingesetzten Komplexkatalysatoren, die Ruthenium sowie mindestens einen Donorliganden, jedoch keinen anionischen Liganden enthalten, primäre Amine gegenüber den im Stand der Technik beschriebenen Verfahren teilweise in deutlich verbesserten Ausbeuten erhalten werden.

Bei der homogen-katalysierten Alkoholaminierung werden die Hydroxylgruppen (-OH) des eingesetzten Alkohols mit Ammoniak zu primären Aminogruppen (-NH₂) umgesetzt, wobei je umgesetzter Hydroxylgruppe ein Molekül Wasser entsteht.

### Edukte

Im erfindungsgemäßen Verfahren werden Alkohole als Edukte eingesetzt, die mindestens eine Hydroxylgruppe (nachfolgend auch als OH-Gruppe bezeichnet) enthalten. Die OH-Gruppe kann dabei in Form der funktionellen Gruppe (-CH₂-OH) (primäre Alkoholgruppe) oder in Form der funktionellen Gruppe (>CH-OH) (sekundäre Alkoholgruppe) vorliegen.

Im erfindungsgemäßen Verfahren werden bevorzugt Edukte eingesetzt, die mindestens eine funktionelle Gruppe der Formel (>CH-OH), das heißt mindestens eine sekundäre Alkoholgruppe, aufweisen. Besonders bevorzugt werden als Edukte Alkohole eingesetzt, die mindestens zwei sekundäre Alkoholgruppen aufweisen.

Als Edukte sind praktisch alle Alkohole geeignet, die die vorstehend genannten Voraussetzungen erfüllen. Die Alkohole können geradkettig, verzweigt oder zyklisch sein. Die Alkohole können darüber hinaus Substituenten tragen, die sich unter den Reaktionsbedingungen der Alkoholaminierung inert verhalten, beispielsweise Alkoxy, Alkenyloxy, Alkylamino, Dialkylamino und Halogene (F, Cl, Br, I). Als Edukte können erfindungsgemäß somit Monoalkohole, Diole, Triole, Polyole und Alkanolamine eingesetzt werden, die bevorzugt mindestens eine funktionelle Gruppe der Formel (>CH-OH) aufweisen.

Erfindungsgemäß einzusetzende Monoalkohole sind Alkohole, die nur eine Hydroxylgruppe aufweisen. Erfindungsgemäß einzusetzende Diole, Triole und Polyole sind Alkohole, die zwei, drei oder mehrere Hydroxylgruppen aufweisen. Erfindungsgemäß einzusetzende Alkanolamine sind Verbindungen, die mindestens eine Hydroxylgruppe und mindestens eine weitere primäre, sekundäre, oder tertiäre Aminogruppe aufweisen.

Geeignete Alkohole sind beispielsweise solche der allgemeinen Formel (XX): 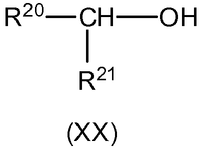 in der
- R²⁰ und R²¹: unabhängig voneinander ausgewählt sind aus der Gruppe bestehend aus Wasserstoff, unsubstituiertem oder zumindest monosubstituiertem C₁-C₃₀-Alkyl, C₅-C₁₀-Cycloalkyl, C₅-C₁₀-Heterocyclyl, C₅-C₁₄-Aryl und C₅-C₁₄-Heteroaryl oder R²⁰ und R²¹ zusammen mit dem Kohlenstoffatom, an das sie gebunden sind, ein fünf- bis zwölfgliedriges unsubstituiertes oder zumindest monosubstituiertes Ringsystem ausbilden,
wobei die Substituenten ausgewählt sind aus der Gruppe bestehend aus F, Cl, Br, OH, OR²², CN, NH₂, NHR²², N(R²²)₂, COOH, COOR²², C(O)NH₂, C(O)NHR²², C(O)N(R²²)₂, C₁-C₁₀-Alkyl, C₅-C₁₀-Cycloalkyl, C₅-C₁₀-Heterocyclyl, C₅-C₁₄-Aryl und C₅-C₁₄-Heteroaryl.
wobei R²² ausgewählt ist aus C₁-C₁₀-Alkyl und C₅-C₁₀-Aryl.

Für den Fall, dass R²⁰ und R²¹ zusammen mit dem Kohlenstoffatom, an das sie gebunden sind, ein Ringsystem ausbilden, ist das Ringsystem bevorzugt ausgewählt aus der Gruppe bestehend aus unsubstituiertem oder zumindest monosubstituiertem C₅-C₁₄-Cycloalkyl, C₅-C₁₄-Heterocyclyl, C₅-C₁₄-Aryl und C₅-C₁₄-Heteroaryl, wobei die Substituenten die vorstehend genannten Bedeutungen aufweisen.

Besonders bevorzugte Ringsysteme sind ausgewählt aus der Gruppe bestehend aus unsubstituiertem oder zumindest monosubstituiertem Cyclopentyl, Cyclohexyl, Cycloheptyl, Cyclooktyl, Phenyl, Naphtyl, Anthryl und Phenanthryl.

Geeignet sind beispielsweise die folgenden Alkohole: Methanol, Ethanol, n-Propanol, n-Butanol, iso-Butanol, n-Pentanol, n-Hexanol, n-Heptanol, n-Oktanol, n-Nonanol, 2-Ethylhexanol, Tridekanol, Stearylalkohol, Palmitylalkohol, Benzylalkohol, 2-Phenylethanol, 2-(p-Methoxyphenyl)ethanol, Furfurylalkohol, 2-(3,4-Dimethoxyphenyl)ethanol, Hydroxymethylfurfural, Allylalkohol, Propargylalkohol, Milchsäure und Serin.

Bevorzugt als Edukte sind Alkohole, die mindestens zwei Hydroxylgruppen aufweisen.

Besonders bevorzugt als Edukte sind Alkohole, die mindestens zwei funktionelle Gruppen der Formel (>CH-OH) (sekundäre Alkoholgruppe) aufweisen.

Beispiele für Diole, die als Edukte in das erfindungsgemäße Verfahren eingesetzt werden können, sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 1,2-Butandiol (1,2-Butylenglykol), 2,3-Butandiol, 2-Methyl-1,3-propandiol, 2,2-Dimethyl-1,3-propandiol (Neopentylglykol), 1,5-Pentandiol, 1,2-Pentandiol, 1,6-Hexandiol, 1,2-Hexandiol, 1,7-Heptandiol, 1,2-Heptandiol, 1,8-Oktandiol, 1,2-Oktandiol, 1,9-Nonandiol, 1,2-Nonandiol, 2,4-Dimethyl-2,5-hexandiol, Hydroxypivalinsäureneopentylglykolester, Diethylenglykol, Triethylenglykol, 2-Buten-1,4-diol, 2-Butin-1,4-diol, Poylyethlenglykole, Polypropylenglykole wie 1,2-Polypropylenglykol und 1,3 Polypropylenglykol, Polytetrahydrofuran, Diethanolamin, 1,4-Bis-(2-hydroxyethyl)piperazin, Diisopropanolamin, N-Butyldiethanolamin, 1,10-Dekandiol, 1,12-Dodekandiol, 2,5-(Dimethanol)-furan, 1,4-Bis(hydroxymethyl)cyclohexan, C₃₆-Diol (Gemisch aus Isomeren von Alkoholen der Summenformel (C₃₆H₇₄O₂)) und N-Methyldiethanolamin, Isosorbid (1,4:3,6 - Dianhydroglucitol), Isomannid (1,4 : 3,6 -Dianhydromannitol, Diisopropanol-p-Toluidin, N,N-Di-(2-hydroxyethyl)aniline, Glucarsäure-1,4 : 6,3-dilacton (CAS 826-91-5), Diisopropanolamin. 2,5-(Dimethanol)-furan wird auch als 2,5-Bis(hydroxymethyl)furan bezeichnet.

Insbesondere bevorzugte Diole, die mindestens zwei funktionelle Gruppen der Formel (>CH-OH) (sekundäre Alkoholgruppe) aufweisen sind ausgewählt aus Glucarsäure-1,4 : 6,3-dilacton, Isosorbid, Isomannid, Isoidid, Mannitol, Sorbitol und Galactitol.

Als Edukte können alle bekannten Triole oder Polyole eingesetzt werden, die mindestens eine funktionelle Gruppe der Formel (-CH₂-OH) oder (>CH-OH) aufweisen, besonders bevorzugt Triole mit mindestens zwei funktionellen Gruppen der Formel (-CH₂-OH) und/oder (>CH-OH). Beispiele für Triole oder Polyole, die als Edukte in das erfindungsgemäße Verfahren eingesetzt werden können, sind Glycerin, Trimethylolpropan, Triisopropanolamin, Triethanolamin, Polyvinylalkohol, 2,2-Bis(hydroxymethyl)-1,3-propandiol (Pentaerythrit), Sorbit, Inositol, Kohlenhydrate, Zucker, Zuckeralkohole und Polymere wie beispielsweise Glucose, Mannose, Fructose, Ribose, Desoxyribose, Galactose, N-Acetyl-Glucosamin, Fucose, Rhamnose, Saccharose, Lactose, Cellobiose, Maltose und Amylose, Cellulose, Stärke und Xanthan.

Bevorzugt sind Triole oder Polyole, die mindestens zwei sekundäre Alkoholgruppen (>CH-OH) aufweisen.

Das erfindungsgemäße Verfahren ist insbesondere zur Aminierung von Alkoholen geeignet, die thermisch instabil sind und beim Verdampfen, auch unter vermindertem Druck, zur Zersetzung neigen.

Besonders bevorzugte Alkohole sind ausgewählt aus der Gruppe bestehend aus Zuckern, Zuckeralkoholen und den daraus durch chemische Umsetzung (z. B. durch Dehydratisierung) ableitbaren Derivaten wie Desoxyzucker und C- oder O-Glycosiden.

Ganz besonders bevorzugte Alkohole sind ausgewählt aus der Gruppe bestehend aus Isosorbid, Isomannid, Isoidid, Mannitol, Glucarsäure-1,4:6,3-dilacton, Sorbitol und Galactitol.

Am meisten bevorzugte Alkohole sind ausgewählt aus der Gruppe bestehend aus Isosorbid und Isomannid. Unter den Alkoholen Isosorbid und Isomannid ist Isosorbid bevorzugt.

Als Edukte können darüber hinaus auch alle bekannten Alkanolamine eingesetzt werden, die mindestens eine OH-Gruppe, bevorzugt eine primäre oder sekundäre Hydroxylgruppe und mindestens eine primäre Aminogruppe (-NH₂) aufweisen. Die Alkanolamine werden im Rahmen der Erfindung mit unter die als Edukte einzusetzenden Alkohole gefasst. Beispiele für Alkanolamine, die als Edukte in das erfindungsgemäße Verfahren eingesetzt werden können, sind Monoaminoethanol, 3-Aminopropan-1-ol, 2-Aminopropan-1-ol, 4-Aminobutan-1-o|, 2-Aminobutan-1-ol, 3-Aminobutan-1-ol, 5-Aminopentan-1-ol, 2-Aminopentan-1-ol, 6-Aminohexan-1-ol, 2-Aminohexan-1ol, 7-Aminoheptan-1-ol, 2-Aminoheptan-1-ol, 8-Aminooctan-1-ol, 2-Aminooctan-1-ol, N-(2-Hydroxyethyl)anilin, 2-(2-Aminoethoxy)ethanol, N-(2-Hydroxyethyl)-1,3-propandiamin und Aminodiethylenglykol (2-(2-Aminoethoxy)ethanol).

Bevorzugt sind Alkanolamine, die mindestens eine primäre Hydroxylgruppe (-CH₂-OH) und mindestens eine primäre Aminogruppe der Formel (-CH₂-NH₂) aufweisen.

Insbesondere bevorzugte Alkanolamine sind Monoaminoethanol, 3-Aminopropan-1-ol, 2-Aminopropan-1-ol, 4-Aminobutan-1-ol und 2-(2-Aminoethoxy)ethanol.

Im erfindungsgemäßen Verfahren kann genau ein Alkohol eingesetzt werden. Es ist auch möglich, Mischungen aus zwei oder mehreren Alkoholen einzusetzen.

### Komplexkatalysator

Im erfindungsgemäßen Verfahren wird mindestens ein Komplexkatalysator eingesetzt, der Ruthenium sowie mindestens einen Donorliganden, jedoch keine anionischen Liganden enthält.

Der Donorligand ist bevorzugt mindestens bidental.

Unter Dentalität (auch als Zähnigkeit bezeichnet) wird die Anzahl der Koordinationsstellen verstanden, die der Donorligand am Zentralatom (Ruthenium) besetzen kann.

Monodentale (einzähnige) Liganden können nur eine Koordinationsstelle des Zentralatoms besetzen.

Bidentale (zweizähnige) Liganden können zwei und tridentale (dreizähnige) Liganden können drei Koordinationsstellen des Zentralatoms besetzen.

Das erfindungsgemäße Verfahren wird homogen-katalysiert in Gegenwart mindestens eines Komplexkatalysators der allgemeinen Formel (I) durchgeführt: 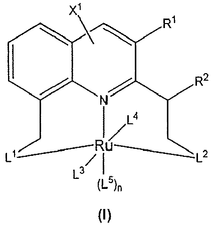 in der
- L¹ und L²: sind unabhängig voneinander PR^{a}R^{b} oder NR^{a}R^{b};
- L³, L⁴ und L⁵: sind unabhängig voneinander einzähnige Zweielektronendonoren ausgewählt sind aus der Gruppe CO und PR^{a}R^{b}R^{c};
- n: ist eine ganze Zahl 0 oder 1;
- R¹ und R²: sind beide Wasserstoff oder bilden gemeinsam mit den Kohlenstoffatomen, an die sie gebunden sind, einen Phenylring, der zusammen mit der Chinolinyleinheit der Formel I eine Acridinyleinheit bildet;
- R, R^{a}, R^{b} und R^{c}: sind unabhängig voneinander unsubstituiertes oder zumindest monosubstituiertes C₁-C₁₀-Alkyl, C₅-C₁₀-Cycloalkyl, C₅-C₁₀-Heterocyclyl, C₅-C₁₀-Aryl oder C₅-C₁₀-Heteroaryl, wobei die Substituenten ausgewählt sind aus der Gruppe bestehend aus F, Cl, Br, OH, CN, NH₂ und C₁₋C₁₀-Alkyl;
- X¹: stellt ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit dar,
wobei X¹ unabhängig voneinander ausgewählt ist aus der Gruppe bestehend aus Wasserstoff, F, Cl, Br, I, OH, NH₂, NO₂, NC(O)R, C(O)NR₂, OC(O)R, C(O)OR, CN und unsubstituiertem oder zumindest monosubstituiertem C₁₋C₁₀-Alkoxy, C₁-C₁₀-Alkyl, C₅-C₁₀-Cycloalkyl, C₅-C₁₀-Heterocyclyl, C₅-C₁₀-Aryl und C₅-C₁₀-Heteroaryl,
wobei die Substituenten ausgewählt sind aus der Gruppe bestehend aus F, Cl, Br, OH, CN, NH₂ und C₁-C₁₀-Alkyl.

Der Komplexkatalysator kann neutral, einfach oder zweifach positiv geladen sein, bevorzugt ist er neutral.

In einer bevorzugten Ausführungsform wird das erfindungsgemäße Verfahren in Gegenwart mindestens eines Komplexkatalysators durchgeführt, wobei R¹ und R² beide Wasserstoff sind und der Komplexkatalysator ein Katalysator der Formel (IV) ist: 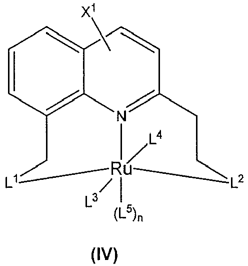 in der X¹, L¹, L², L³, L⁴, L⁵ und n die vorstehend genannten Bedeutungen aufweisen, wobei die vorstehend genannten Bevorzugungen entsprechend gelten.

In einer weiteren besonders bevorzugten Ausführungsform wird das erfindungsgemäße Verfahren in Gegenwart mindestens eines Komplexkatalysator durchgeführt, wobei R¹ und R² gemeinsam mit den Kohlenstoffatomen, an die sie gebunden sind einen Phenylring bilden, der zusammen mit der Chinolinyleinheit der Formel (I) eine Acridinyleinheit bildet und der Komplexkatalysator ein Katalysator der Formel (V) ist: 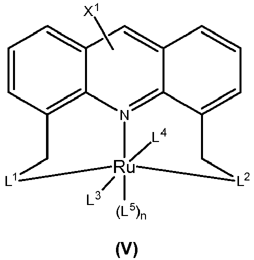 in der X¹, L¹, L², L³, L⁴, L⁵ und n die vorstehend genannten Bedeutungen aufweisen, wobei die vorstehend genannten Bevorzugungen entsprechend gelten.

In einer weiteren bevorzugten Ausführungsform wird das erfindungsgemäße Verfahren in Gegenwart mindestens eines Komplexkatalysators aus der Gruppe bestehend aus Katalysatoren der Formel (VI), (VII), (VIII), (IX), (X), (XI), (XII) und (XIII) durchgeführt: 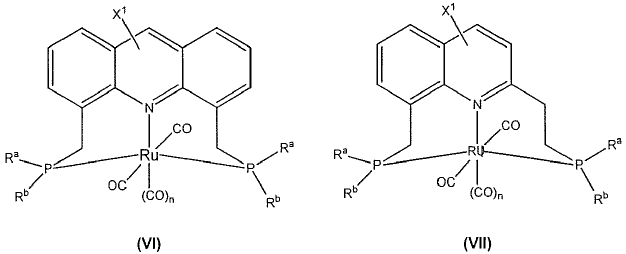 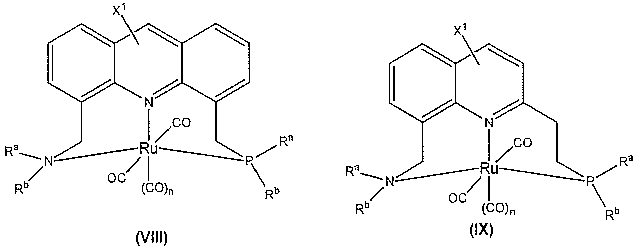 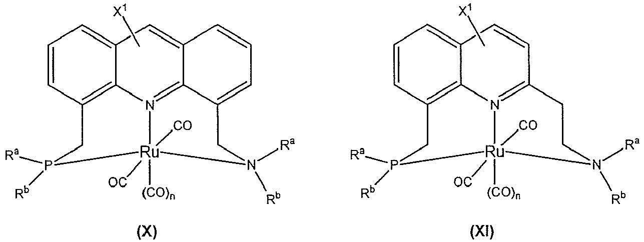 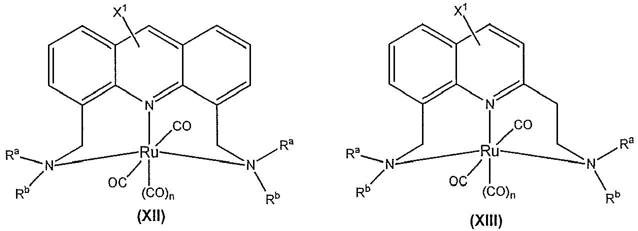 in denen X¹, R^{a}, R^{b} und n die vorstehend genannten Bedeutungen aufweisen, wobei die vorstehend genannten Bevorzugungen entsprechend gelten.

Weiterhin besonders bevorzugt sind Komplexkatalysatoren der Formeln (I), (IV), (V) und (VI) bis (XIII), in denen R^{a} und R^{b} unsubstituiertes Cyclohexyl sind.

In einer ganz besonders bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander unsubstituiertes oder zumindest monosubstituiertes C₁-C₁₀-Alkyl, C₅-C₁₀-Cycloalkyl, C₅-C₁₀-Heterocyclyl, C₅-C₁₀-Aryl oder C₅-C₁₀-Heteroaηl sind;
wobei die Substituenten ausgewählt sind aus der Gruppe bestehend aus F, Cl, Br, OH, CN, NH₂ und C₁-C₁₀-Alkyl;
- n: eine ganze Zahl 0 oder 1 ist;
- X¹: ein, zwei oder drei, Substituenten an einem oder mehreren Atomen der Acridinyleinheit oder ein oder zwei Substituenten an einem oder mehreren Atomen der Chinolinyleinheit darstellt;
wobei X¹ unabhängig voneinander ausgewählt ist aus der Gruppe bestehend aus Wasserstoff, F, Cl, Br, I, OH, NH₂, NO₂, NC(O)R, C(O)NR₂, OC(O)R, C(O)OR, CN und unsubstituiertem C₁-C₁₀-Alkoxy, C₁-C₁₀-Alkyl, C₅-C₁₀-Cycloalkyl, C₅-C₁₀-Hetero-cyclyl, C₅-C₁₀-Aryl und C₅-C₁₀-Heteroaryl,
wobei R ausgewählt ist aus der Gruppe bestehend aus H und C₁-C₁₀-Alkyl.

In einer weiteren ganz besonders bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander Methyl, Ethyl, Isopropyl, tert.-Butyl, Cyclohexyl, Cyclopentyl, Phenyl oder Mesityl sind;
- n: eine ganze Zahl 0 oder 1 ist;
- X¹: Wasserstoff ist.

In einer insbesondere bevorzugten Ausführungsform wird das erfindungsgemäße Verfahren in Gegenwart eines Komplexkatalysators der Formel (XIVa) durchgeführt: 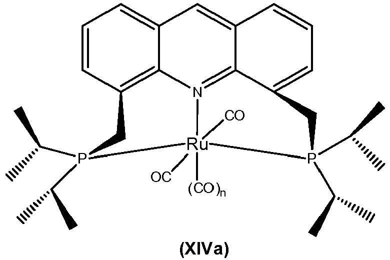 in der n eine ganze Zahl 0 oder 1 ist.

In einer weiteren insbesondere bevorzugten Ausführungsform wird das erfindungsgemäße Verfahren in Gegenwart eines Komplexkatalysators der Formel (XIVb) durchgeführt. 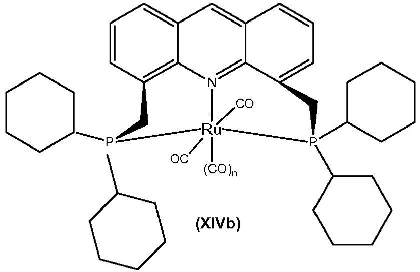 in der n eine ganze Zahl 0 oder 1 ist.

In einer besonders bevorzugten Ausführungsform wird das erfindungsgemäße Verfahren in Gegenwart mindestens eines Komplexkatalysators der Formeln (I), (IV), (V) und (VI) bis (XIII) sowie XIVa)) oder (XIVb)) durchgeführt, in denen n gleich 0 ist.

Im Rahmen der vorliegenden Erfindung werden unter C₁-C₃₀ bzw. C₁-C₁₀-Alkyl verzweigte, unverzweigte, gesättigte und ungesättigte Gruppen verstanden. Bevorzugt sind Alkylgruppen mit 1 bis 6 Kohlenstoffatomen (C₁-C₆-Alkyl). Mehr bevorzugt sind Alkylgruppen mit 1 bis 4 Kohlenstoffatomen (C₁-C₄-Alkyl).

Beispiele für gesättigte Alkylgruppen sind Methyl, Ethyl, n-Propyl, Isopropyl, n-Butyl, Isobutyl, sec.-Butyl, tert.-Butyl, Amyl und Hexyl.

Beispiele für ungesättigte Alkylgruppen (Alkenyl, Alkinyl) sind Vinyl, Allyl, Butenyl, Ethinyl und Propinyl.

Die C₁-C₁₀-Alkylgruppe kann unsubstituiert oder, mit einem oder mehreren Substituenten ausgewählt aus der Gruppe F, Cl, Br, Hydroxy (OH), C₁-C₁₀-Alkoxy, C₅-C₁₀-Aryloxy, C₅-C₁₀-Alkylaryloxy, C₅-C₁₀-Heteroaryloxy enthaltend mindestens ein Heteroatom ausgewählt aus N, O, S, Oxo, C₃-C₁₀-Cycloalkyl, Phenyl, C₅-C₁₀-Heteroaryl enthaltend mindestens ein Heteroatom ausgewählt aus N, O, S, C₅-C₁₀-Heterocyclyl enthaltend mindestens ein Heteroatom ausgewählt aus N, O, S, Naphthyl, Amino, C₁-C₁₀-Alkylamino, C₅-C₁₀-Arylamino, C₅-C₁₀-Heteroarylamino enthaltend mindestens ein Heteroatom ausgewählt aus N, O, S, C₁-C₁₀-Dialkylamino, C₁₀-C₁₂-Diarylamino, C₁₀-C₂₀Alkylarylamino, C₁-C₁₀-Acyl, C₁-C₁₀-Acyloxy, NO₂, C₁-C₁₀-Carboxy, Carbamoyl, Carboxamid, Cyano, Sulfonyl, Sulfonylamino, Sulfinyl, Sulfinylamino, Thiol, C₁-C₁₀-Alkylthiol, C₅-C₁₀-Arylthiol oder C₁-C₁₀-Alkylsulfonyl substituiert sein.

Unter C₅-C₁₀-Cycloalkyl werden vorliegend gesättigte, ungesättigte monozyklische und polyzyklische Gruppen verstanden. Beispiele für C₅-C₁₀-Cycloalkyl sind Cyclopentyl, Cyclohexyl oder Cycloheptyl. Die Cycloalkylgruppen können unsubstituiert oder substituiert sein mit einem oder mehreren Substituenten, wie sie vorstehend zu der Gruppe C₁-C₁₀-Alkyl definiert wurde.

Im Rahmen der vorliegenden Erfindung wird unter C₅-C₁₀-Aryl ein aromatisches Ringsystem mit 5 bis 10 Kohlenstoffen verstanden. Das aromatische Ringsystem kann monozyklisch oder bizyklisch sein. Beispiele für Arylgruppen sind Phenyl, Naphthyl wie 1-Naphthyl und 2-Naphthyl. Die Arylgruppe kann unsubstituiert oder substituiert sein, mit einem oder mehreren Substituenten wie vorstehend unter C₁-C₁₀-Alkyl definiert.

Im Rahmen der vorliegenden Erfindung wird unter C₅-C₁₀-Heteroaryl ein heteroaromatisches System verstanden, das mindestens ein Heteroatom, ausgewählt aus der Gruppe N, O und S, enthält. Die Heteroarylgruppen können monozyklisch oder bizyklisch sein. Für den Fall, dass Stickstoff ein Ringatom ist, umfasst die vorliegende Erfindung auch N-Oxide der stickstoffenthaltenden Heteroaryle. Beispiele für Heteroaryle sind Thienyl, Benzothienyl, 1-Naphthothienyl, Thianthrenyl, Furyl, Benzofuryl, Pyrrolyl, Imidazolyl, Pyrazolyl, Pyridyl, Pyrazinyl, Pyrimidinyl, Pyridazinyl, Indolyl, Isoindolyl, Indazolyl, Purinyl, Isoquinolinyl, Quinolinyl, Acridinyl, Naphthyridinyl, Quinoxalinyl, Quinazolinyl, Cinnolinyl, Piperidinyl, Carbolinyl, Thiazolyl, Oxazolyl, Isothiazolyl, Isoxazolyl. Die Heteroarylgruppen können unsubstituiert oder substituiert sein, mit einem oder mehreren Substituenten, die vorstehend unter C₁-C₁₀-Alkyl definiert wurden.

Im Rahmen der vorliegenden Erfindung werden unter C₅-C₁₀-Heterocyclyl fünf- bis zehngliedrige Ringsysteme verstanden, die mindestens ein Heteroatom aus der Gruppe N, O und S enthalten. Die Ringsysteme können mono- oder bizyklisch sein. Beispiele für geeignete heterozyklische Ringsysteme sind Piperidinyl, Pyrrolidinyl, Pyrrolinyl, Pyrazolinyl, Pyrazolidinyl, Morpholinyl, Thiomorpholinyl, Pyranyl, Thiopyranyl, Piperazinyl, Indolinyl, Dihydrofuranyl, Tetrahydrofuranyl, Dihydrothiophenyl, Tetrahydrothiophenyl, Dihydropyranyl und Tetrahydropyranyl.

### Alkoholaminierung

Der bei dem erfindungsgemäßen Verfahren zur Alkoholaminierung eingesetzte Komplexkatalysator weist keine anionischen Liganden auf. Im Rahmen der vorliegenden Erfindung werden unter anionischen Liganden Verbindungen verstanden, die mindestens eine negative Ladung tragen. Beispiele für anionische Liganden, die eine negative Ladung tragen (monoanionische Liganden) sind H (Hydrid), Halogenidanionen, Carboxylate, Sulfonate, CN (Cyanidanion) und OH (Hydroxidanion). Solche anionischen Liganden sind im Komplexkatalysator nicht enthalten.

In einer weiteren Ausführungsform weist der Komplexkatalysator keine ionischen Liganden, das heißt weder anionische noch kationische Liganden, auf. Unter anionischen bzw. kationischen Liganden werden erfindungsgemäß nur solche Liganden verstanden, die direkt an das Ru-Zentralatom des Komplexkatalysators koordiniert sind. Gegenionen, insbesondere Anionen, die nicht direkt an das Ru-Zentralatom koordiniert sind, stellen erfindungsgemäß keine anionischen oder kationischen Liganden dar.

Neben dem Komplexkatalysator, der keine anionischen Liganden enthält, können bei der Alkoholaminierung auch Komplexkatalysatoren zugegen sein, die einen oder mehrere anionische Liganden aufweisen. Der erfindungsgemäße Komplexkatalysator, der keine ionischen Liganden, bevorzugt keine anionischen Liganden, aufweist, wird im Rahmen der vorliegenden Erfindung auch als Komplexkatalysator (KKe) bezeichnet. Komplexkatalysatoren, die einen oder mehrere anionische Liganden aufweisen, werden im Rahmen der vorliegenden Erfindung auch als Komplexkatalysator (KKs) bezeichnet.

Im erfindungsgemäßen Verfahren kann genau ein Komplexkatalysator (KKe) eingesetzt werden. Es ist auch möglich, Mischungen aus zwei oder mehreren Komplexkatalysatoren (KKe) einzusetzen.

Für den Fall, dass die erfindungsgemäße Alkoholaminierung in Gegenwart eines Katalysators durchgeführt wird, der eine Mischung eines Komplexkatalysators (KKe) und eines Komplexkatalysators (KKs) enthält, stellt der Komplexkatalysator (KKe) mengenmäßig den Hauptteil der Mischung dar, bezogen auf die Gesamtmenge der in der Mischung enthaltenen Komplexkatalysatoren (KKe) und (KKs), in deren Gegenwart das erfindungsgemäße Verfahren durchgeführt wird.

Unter Hauptteil wird dabei verstanden, dass für den Fall, dass neben dem oder den erfindungsgemäßen Komplexkatalysatoren (KKe) auch Komplexkatalysatoren (KKs) zugegen sind, das Mengenverhältnis dieser Komplexkatalysatoren (KKe) und (KKs) zueinander dem Mengenverhältniskoeffizienten Mᵥ = [Gesamtmenge der Komplexkatalysatoren (KKe), in deren Gegenwart die Alkoholaminierung durchgeführt wird] / [Gesamtmenge der Komplexkatalysatoren (KKs), in deren Gegenwart die Alkoholaminierung durchgeführt wird] entspricht und Mᵥ im Allgemeinen > 1, bevorzugt > 5, mehr bevorzugt > 9, besonders bevorzugt > 20. Die Gesamtmengen des Komplexkatalysators (KKe) und (KKs) werden jeweils in Mol-% gemessen.

Der erfindungsgemäße Komplexkatalysator kann sowohl direkt in seiner aktiven Form eingesetzt werden als auch ausgehend von rutheniumhaltigen Katalysatorvorstufen unter Zugabe des entsprechenden Liganden erst unter den Reaktionsbedingungen erzeugt werden. Für den Fall, dass der Komplexkatalysator direkt in seiner aktiven Form eingesetzt wird, wird dieser in einem der eigentlichen Alkoholaminierung vorgelagerten Verfahrensschritt hergestellt.

Zur Herstellung des Komplexkatalysators in einem vorgelagerten Verfahrensschritt wird mindestens ein Donorligand der allgemeinen Formel (la) 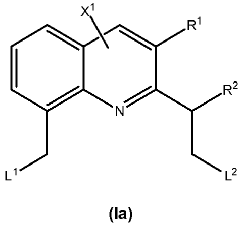 eingesetzt, in der X¹, L¹, L², R¹ und R² die vorstehend für die Komplexkatalysatoren der Formeln (I), (IV), (V) und (VI) bis (XIII) sowie (XIVa) und (XIVb) beschriebenen Bedeutungen aufweisen, wobei die dort beschriebenen Bevorzugungen entsprechend gelten.

Der Donorligand (la), eine rutheniumhaltige Katalysatorvorstufe und ein Lösungsmittel werden, vorzugsweise bei Raumtemperatur (20°C), in einen Autoklaven eingebracht. Der Donorligand (la) wird bevorzugt in äquimolaren Mengen bezogen auf das in der ruthemiumhaltigen Katalysatorvorstufe enthaltene Ruthenium eingesetzt. Es ist auch möglich, den Donorligand (la) im Überschuss oder im Unterschuss einzusetzen.

Bevorzugt erfolgt das Einbringen der vorstehend beschriebenen Komponenten unter Schutzgasatmosphäre, beispielsweise unter Stickstoff oder Argon. Als Lösungsmittel können polare oder unpolare Lösungsmittel eingesetzt werden. Bevorzugt sind unpolare Lösungsmittel wie beispielsweise Benzol, Toluol oder Xylole (1,2-Dimethylbenzol, 1,3-Dimethylbenzol, 1,4-Dimethylbenzol) sowie Mischungen dieser unpolaren Lösungsmittel. Als unpolares Lösungsmittel ist Toluol bevorzugt. Die Lösungsmittel werden bevorzugt in absolutierter Form eingesetzt. Zur Bildung des Komplexkatalysators wird der Autoklav auf Temperaturen im Bereich von 50 bis 150°C, bevorzugt im Bereich von 80 bis 120°C und besonders bevorzugt im Bereich von 90 bis 110°C erwärmt. Die Reaktionszeit bei diesen Temperaturen beträgt im Allgemeinen 1 bis 100 Stunden, bevorzugt 5 bis 50 Stunden, besonders bevorzugt 10 bis 30 Stunden und insbesondere 18 bis 22 Stunden.

Die Bildung des Komplexkatalysators kann dadurch unterstützt werden, dass man den Autoklaven vor dem Erwärmen, vorzugsweise bei Raumtemperatur, mit Kohlenmonoxid oder Synthesegas bis zu einem Druck im Bereich von 20 bis 60 bar, bevorzugt 30 bis 50 bar und besonders bevorzugt im Bereich von 35 bis 45 bar beaufschlagt. Dies ist jedoch nicht zwingend erforderlich.

Nachfolgend wird der Autoklav abgekühlt und entspannt und das Lösungsmittel wird durch Vakuumdestillation entfernt, wodurch der Komplexkatalysator als Feststoff erhalten wird. Der Komplexkatalysator kann nachfolgend weiter aufgearbeitet werden, beispielsweise durch Waschen mit einem weiteren Lösungsmittel, bevorzugt mit einem polaren Lösungsmittel wie Tetrahydrofuran (THF).

Es ist auch möglich, das Lösungsmittel, bevorzugt das unpolare Lösungsmittel, nicht vollständig zu entfernen und den Komplexkatalysator durch Zugabe eines weiteren Lösungsmittels, bevorzugt eines polaren Lösungsmittels, auszufällen und anschließend durch Filtration abzutrennen.

Unter Synthesegas wird vorliegend ein Gasgemisch verstanden, das im Wesentlichen Kohlenmonoxid (CO) und Wasserstoff (H₂) enthält. Die Volumenanteile CO zu H₂ liegen vorzugsweise im Bereich von 0,8 zu 1,2 bis 1,2 zu 0,8. Bevorzugt liegen die Volumenanteile von CO zu H₂ bei 1 zu 1.

Als ruthemiumhaltige Katalysatorvorstufen sind beispielsweise [Ru₃(CO)₁₂], [Ru(CO)₄(ethylen)], [Ru(COD)(OAc)₂], [Ru(COD)(2-methylallyl)₂], [Ru(CO)₃Cl₂]₂, [RuCl₃*H₂O], [Ru(acetylacetonat)₃], [Ru(PPh₃)₃(CO)(H)Cl], [Ru(PPh₃)₃(CO)Cl₂], [Ru(PPh₃)₃(CO)(H)₂], [Ru(PPh₃)₃Cl₂] und [Ru(COD)Cl₂]₂ geeignet. Selbstverständlich können auch Mischungen aus zwei oder mehreren der vorstehend genannten ruthemiumhaltigen Katalysatorvorstufen eingesetzt werden.

COD bedeutet 1,5-Cyclooctadien. PPh₃ bedeutet Triphenylphosphin.

Gegenstand der vorliegenden Erfindung ist somit auch ein Verfahren zur Herstellung eines Komplexkatalysators (I) durch Umsetzen mindestens einer rutheniumhaltigen Katalysatorvorstufe ausgewählt aus der Gruppe bestehend aus [Ru₃(CO)₁₂], [Ru(CO)₄(ethylen)], [Ru(COD)(OAc)₂], [Ru(COD)(2-methylallyl)₂], [Ru(CO)₃Cl₂]₂, [RuCl₃*H₂O], [Ru(acetylacetonat)_{3]}, [Ru(PPh₃)₃(CO)(H)Cl], [Ru(PPh₃)₃(CO)Cl₂], [Ru(PPh₃)₃(CO)(H)₂], [Ru(PPh₃)₃Cl₂] und [Ru(COD)Cl₂]₂ mit mindestens einem Donorliganden der allgemeinen Formel (la) in Gegenwart eines Lösungsmittels sowie gegebenenfalls in Gegenwart von Kohlenmonoxid oder Synthesegas bei einem Druck im Bereich von 20 bis 60 bar.

Für den Fall, dass eine rutheniumhaltige Katalysatorvorstufe ausgewählt aus der Gruppe bestehend aus rutheniumhaltigen Katalysatorvorstufen [Ru(COD)(OAc)₂], [Ru(COD)(2-methylallyl)₂], [Ru(CO)₃Cl₂]₂, [RuCl₃*H₂O], [Ru(acetylacetonat)₃], [Ru(PPh₃)₃(CO)(H)Cl], [Ru(PPh₃)₃(CO)Cl₂], [Ru(PPh₃)₃(CO)(H)₂], [Ru(PPh₃)₃Cl₂] und [Ru(COD)Cl₂]₂ eingesetzt wird, wird zur Herstellung des Komplexkatalysators bevorzugt Kohlenmonoxid oder Synthesegas aufgepresst.

Für den Fall, dass als rutheniumhaltige Katalysatorvorstufe [Ru₃(CO)₁₂] und/oder [Ru(CO)₄(ethylen)] eingesetzt wird, kann ebenfalls Kohlenmonoxid oder Synthesegas aufgepresst werden. Dies ist jedoch nicht zwingend erforderlich.

Bei der Herstellung des Komplexkatalysators in einem vorgelagerten Verfahrensschritt wird bevorzugt Kohlenmonoxid oder Synthesegas aufgepresst, als Lösungsmittel wird ein unpolares Lösungsmittel, insbesondere Toluol, eingesetzt und als weiteres Lösungsmittel zum Waschen oder Ausfällen wird ein polares Lösungsmittel, insbesondere Tetrahydrofuran eingesetzt.

Es ist auch möglich, den Komplexkatalysator direkt in dem Reaktor der Alkoholaminierung herzustellen. Hierzu werden der Donorligand (la), eine rutheniumhaltige Katalysatorvorstufe und ein Lösungsmittel, vorzugsweise bei Raumtemperatur (20°C) und unter Inertbedingungen, in den Reaktor der Alkoholaminierung eingebracht. Als Lösungsmittel wird dabei bevorzugt ein Lösungsmittel eingesetzt, das nachfolgend auch als Lösungsmittel zur Alkoholaminierung verwendet werden kann. Bevorzugt ist ein polares Lösungsmittel, insbesondere tert-Amylalkohol (2-Methyl-2-butanol).

Als rutheniumhaltige Katalysatorvorstufen können die vorstehend beschriebenen Rutheniumverbindungen eingesetzt werden.

Die Bildung des Komplexkatalysators kann hierbei ebenfalls durch Aufpressen von Kohlenmonoxid oder Synthesegas unterstützt werden, wobei die Ausführungen und Bevorzugungen zur Herstelllung des Komplexkatalysators in einem vorgelagerten Schritt entsprechend gelten.

Der Donorligand (Ia), das polare Lösungsmittel und die rutheniumhaltige Katalysatorvorstufen werden zur Bildung des Komplexkatalysators nachfolgend, analog zur Herstellung des Komplexkatalysators in einem vorgelagerten Schritt, erwärmt. Die Herstellung des Komplexkatalysators in dem Reaktor der Alkoholaminierung, in Gegenwart eines Lösungsmittel, das nachfolgend auch als Lösungsmittel zur Alkoholaminierung dienen kann, hat den Vorteil, dass eine Abtrennung und Aufarbeitung des Komplexkatalysators entfällt.

Es ist auch möglich, den Donorliganden (Ia), eine rutheniumhaltige Katalysatorvorstufe und ein Lösungsmittel, vorzugsweise tert-Amylalkohol, gleichzeitig mit einem Alkohol, der als Edukt zur Alkoholaminierung eingesetzt wird, vorzulegen und den Komplexkatalysator in Gegenwart des Edukts für die Alkoholaminierung herzustellen. Dies kann in Gegenwart oder in Abwesenheit eines Ammoniakpartialdruckes durchgeführt werden. In dieser Ausführungsform wird der Katalysator in situ unter den Reaktionsbedingungen der Alkoholaminierung hergestellt.

Gegenstand der vorliegenden Erfindung ist somit auch ein Verfahren zur Herstellung von primären Aminen durch Alkoholaminierung von Alkoholen unter Wasserabspaltung in einem Reaktor, wobei der Komplexkatalysator (I) in dem Reaktor aus mindestens einer rutheniumhaltigen Katalysatorvorstufe und mindestens einen Donorliganden der allgemeinen Formel (Ia) in Gegenwart eines Lösungsmittels hergestellt wird.

Zur Herstellung des Komplexkatalysators im Reaktor der Alkoholaminierung ist als Lösungsmittel ein polares Lösungsmittel, insbesondere tert-Amylalkohol, bevorzugt und als rutheniumhaltige Katalysatorvorstufe wird vorzugweise [Ru₃(CO)₁₂] und/oder [Ru(CO)₄(ethylen)] eingesetzt. Die Bildung des Komplexkatalysators kann hierbei durch das Aufpressen von Kohlenmonoxid oder Synthesegas unterstützt werden, dies ist jedoch nicht zwingend erforderlich. In einer bevorzugten Ausführungsform wird der Komplexkatalysator ohne Kohlenmonoxid und Synthesegas hergestellt.

Der Donorligand (Ia) wird bevorzugt in äquimolaren Mengen bezogen auf das in der ruthemiumhaltigen Katalysatorvorstufe enthaltene Ruthenium eingesetzt. Es ist auch möglich, den Donorligand (Ia) im Überschuss oder im Unterschuss einzusetzen.

Unter homogen-katalysiert wird im Rahmen der vorliegenden Erfindung verstanden, dass der katalytisch-aktive Teil des Komplexkatalysators zumindest teilweise im flüssigen Reaktionsmedium gelöst vorliegt. In einer bevorzugten Ausführungsform liegen mindestens 90 % des im Verfahren eingesetzten Komplexkatalysators im flüssigen Reaktionsmedium gelöst vor, mehr bevorzugt mindestens 95 %, insbesondere bevorzugt mehr als 99 %, am meisten bevorzugt liegt der Komplexkatalysator vollständig gelöst in flüssigen Reaktionsmedium vor (100%), jeweils bezogen auf die Gesamtmenge im flüssigen Reaktionsmedium.

Der Komplexkatalysator wird in Mengen im Bereich von 0,01 bis 20 Mol%, bevorzugt im Bereich von 0,1 bis 10 Mol% und besonders bevorzugt im Bereich von 0,2 bis 6 Mol% pro Mol OH-Gruppe, die im Edukt der Alkoholaminierung enthalten ist, eingesetzt.

Die Reaktion erfolgt in der Flüssigphase im Allgemeinen bei einer Temperatur von 20 bis 250°C. Bevorzugt wird das erfindungsgemäße Verfahren bei Temperaturen im Bereich von 100 °C bis 220 °C, besonders bevorzugt im Bereich von 140 bis 200°C durchgeführt. Die Flüssigphase kann von einem Lösungsmittel und/oder einem unter den Prozessbedingungen in verflüssigter oder überkritischer Form vorliegenden Gasen, insbesondere Ammoniak gebildet werden.

Die Reaktion kann im Allgemeinen bei einem Gesamtdruck von 0,1 bis 20 MPa absolut, der sowohl der Eigendruck des Lösungsmittels bei der Reaktionstemperatur als auch der Druck eines Gases wie Stickstoff, Argon oder Wasserstoff sein kann, durchgeführt werden. Bevorzugt wird das erfindungsgemäße Verfahren bei einem Gesamtdruck im Bereich von 0,5 bis 15 MPa absolut, insbesondere bevorzugt bei einem Gesamtdruck im Bereich von 1 bis 10 MPa absolut durchgeführt.

In einer bevorzugten Ausführungsform wird das erfindungsgemäße Verfahren in Abwesenheit von Wasserstoff durchgeführt. Unter Abwesenheit von Wasserstoff wird erfindungsgemäß verstanden, dass der Reaktion kein zusätzlicher Wasserstoff zugeführt wird. Gegebenenfalls über andere Gase zugeführte Spuren von Wasserstoff sowie bei der Reaktion gebildete Spuren von Wasserstoff gelten als in Abwesenheit von Wasserstoff gemäß der vorliegenden Erfindung. Synthesegas, das gegebenenfalls zu Beginn der Reaktion zur Bildung des Komplexkatalysators in den Reaktor eingebracht wird, gilt ebenfalls als in Abwesenheit von Wasserstoff gemäß der vorliegenden Erfindung.

Die mittlere Reaktionszeit beträgt im Allgemeinen 15 Minuten bis 100 Stunden.

Das Aminierungsmittel (Ammoniak) kann bezüglich der zu aminierenden Hydroxylgruppen in stöchiometrischen, unterstöchiometrischen oder überstöchiometrischen Mengen eingesetzt werden. Als Aminierungsmittel können Ammoniak selbst in Reinform oder als Lösung oder Mischung oder eine Verbindung, die unter den Reaktionsbedingungen Ammoniak freisetzt, eingesetzt werden.

In einer bevorzugten Ausführungsform wird Ammoniak mit einem 1- bis 250-fachen, bevorzugt mit einem 2- bis 100-fachen, insbesondere mit einem 1,5- bis 30-fachen molaren Überschuss pro Mol im Edukt umzusetzender Hydroxylgruppen eingesetzt. Es sind auch höhere Überschüsse an Ammoniak möglich.

Das erfindungsgemäße Verfahren kann sowohl in einem Lösungsmittel als auch ohne Lösungsmittel durchgeführt werden. Als Lösungsmittel eignen sich polare und unpolare Lösungsmittel, die in Reinform oder in Mischungen eingesetzt werden können. Beispielsweise kann im erfindungsgemäßen Verfahren nur ein unpolares oder ein polares Lösungsmittel eingesetzt werden. Es ist auch möglich, Mischungen von zwei oder mehr polaren Lösungsmitteln oder Mischungen von zwei oder mehr unpolaren Lösungsmitteln oder Mischungen aus einem oder mehr polaren mit einem oder mehr unpolaren Lösungsmitteln einzusetzen. Auch das Produkt kann als Lösungsmittel in Reinform oder in Mischungen mit polaren oder mit unpolaren Lösungsmittel eingesetzt werden.

Als unpolare Lösungsmittel eignen sich beispielsweise gesättigte und ungesättigte Kohlenwasserstoffe wie Hexan, Heptan, Oktan, Cyclohexan, Benzol, Toluol, Xylol und Mesitylen, und lineare und zyklische Ether wie THF, Diethylether, 1,4-Dioxan, MTBE (tert-Butylmethylether), Diglyme und 1,2-Dimethoxyethan. Bevorzugt werden Toluol, Xylole oder Mesitylen eingesetzt.

Als polare Lösungsmittel eignen sich beispielsweise Wasser, Dimethylformamid, Formamid, tert-Amylalkohol und Acetonitril. Bevorzugt wird tert-Amylalkohol eingesetzt. Wasser kann sowohl vor der Reaktion zugesetzt werden, bei der Reaktion als Reaktionswasser entstehen oder auch nach der Reaktion zusätzlich zum Reaktionswasser zugesetzt werden.

Für die Umsetzung in der Flüssigphase leitet man Ammoniak, den oder die Alkohole, gegebenenfalls zusammen mit einem oder mehreren Lösungsmitteln, zusammen mit dem Komplexkatalysator in einen Reaktor.

Die Zuleitung von Ammoniak, Edukt (Alkohol), gegebenenfalls Lösungsmittel und Komplexkatalysator kann dabei simultan oder getrennt voneinander erfolgen. Die Reaktion kann dabei kontinuierlich, in semibatch-Fahrweise, in batch-Fahrweise, rückvermischt in Produkt als Lösungsmittel oder im geraden Durchgang nicht rückvermischt durchgeführt werden.

Für das erfindungsgemäße Verfahren können prinzipiell alle Reaktoren eingesetzt werden, welche für gas/flüssig-Reaktionen unter der gegebenen Temperatur und dem gegebenen Drucks grundsätzlich geeignet sind. Geeignete Standardreaktoren für gas/flüssig- und für flüssig/flüssig-Reaktionssysteme sind beispielsweise in K.D.

Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Bei der Aminierungsreaktion wird mindestens eine primäre oder sekundäre Hydroxylgruppe des Edukts mit Ammoniak zu einer primären Aminogruppe umgesetzt, wobei sich jeweils ein Mol Reaktionswasser pro Mol umgesetzter Hydroxylgruppe bildet.

So bilden sich bei der Umsetzung von Alkanolaminen, die nur eine primäre oder sekundäre Hydroxylgruppe aufweisen, die entsprechenden Diamine. Die Umsetzung von Monoaminoethanol führt somit zum entsprechenden 1,2-Diaminoethan.

Bei der Reaktion von Edukten, die zwei primäre oder sekundäre Hydroxylgruppen aufweisen (Diole), werden bevorzugt beide Hydroxylgruppen mit Ammoniak zu den entsprechenden primären Diaminen umgesetzt. Die Umsetzung von 1,2-Ethylenglykol führt somit zum entsprechenden 1,2-Diaminoethan.

Bei der Reaktion von Edukten, die drei primäre oder sekundäre Hydroxylgruppen aufweisen (Triole), werden zwei oder drei Hydroxylgruppen mit Ammoniak zu den entsprechenden primären Diaminen oder Triaminen umgesetzt. Die Bildung von Diaminen oder Triaminen kann dabei durch die Menge des eingesetzten Ammoniaks und durch die Reaktionsbedingungen gesteuert werden. Die Umsetzung von Glycerin führt somit zum entsprechenden 1,2-Diaminopropanol oder zum 1,2,3-Triaminopropan.

Bei der Reaktion von Edukten, die mehr als drei Hydroxylgruppen aufweisen (Polyole), werden zwei, drei oder mehr Hydroxylgruppen mit Ammoniak zu den entsprechenden primären Diaminen, Triaminen oder Polyaminen umgesetzt. Die Bildung der entsprechenden primären Diaminen, Triaminen oder Polyaminen kann dabei durch die Menge des eingesetzten Ammoniaks und durch die Reaktionsbedingungen gesteuert werden.

Die erfindungsgemäße Aminierungsreaktion wird nachfolgend exemplarisch anhand der besonders bevorzugten Umsetzung der Dianhydrohexitole Isomannid und Isosorbid beschrieben.

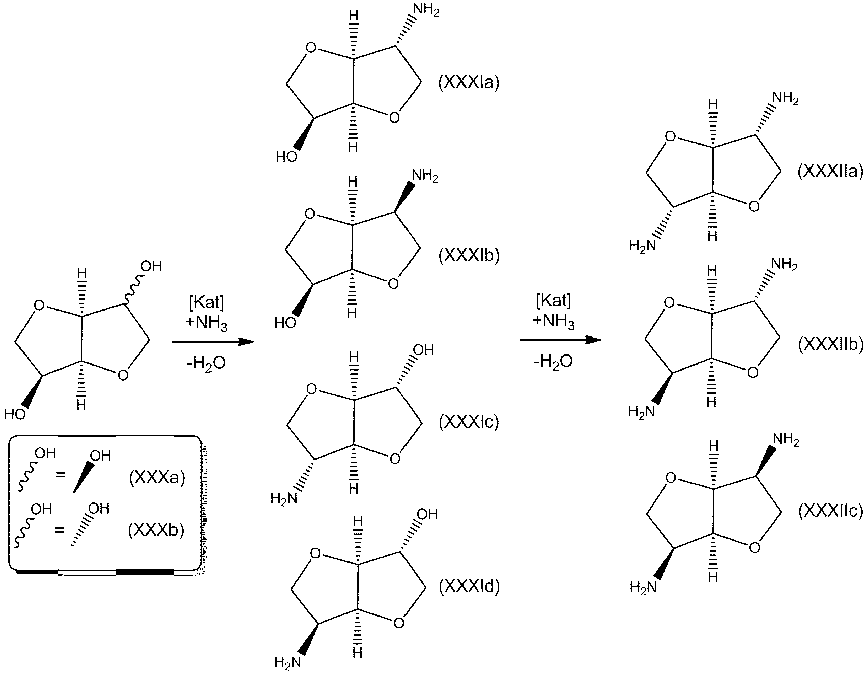

XXXa ist Isomannid. XXXb ist Isosorbid. Bei der Alkoholaminierung entsteht in einem ersten Schritt zunächst ein Gemisch der entsprechenden Monoaminierungsprodukte (Aminoalkohole (XXXIa-XXXId), die nachfolgend zu den entsprechenden Diaminierungsprodukten umgesetzt werden können (Diaminoisoidid (XXXIIa), Diaminoisosorbid (XXXIIb) und Diaminoisomannid (XXXIIc).

Der bei der Umsetzung entstehende Reaktionsaustrag enthält im Allgemeinen die entsprechenden Aminierungsprodukte, gegebenenfalls das eine oder die mehreren Lösungsmittel, den Komplexkatalysator, gegebenenfalls nicht umgesetzte Edukte und Ammoniak sowie das entstandene Reaktionswasser.

Aus dem Reaktionsaustrag werden der gegebenenfalls vorhandene überschüssige Ammoniak, das gegebenenfalls vorhandene Lösungsmittel, der Komplexkatalysator und das Reaktionswasser entfernt. Das erhaltene Aminierungsprodukt kann weiter aufgearbeitet werden. Der überschüssige Ammoniak, der Komplexkatalysator, gegebenenfalls das oder die Lösungsmittel und gegebenenfalls nicht umgesetzte Edukte können in die Aminierungsreaktion zurückgeführt werden.

Wird die Aminierungsreaktion ohne Lösungsmittel durchgeführt, so ist der Komplexkatalysator nach der Reaktion im Produkt, d.h. den Aminierungsprodukten, gelöst. Dieser kann im Produkt verbleiben oder durch eine geeignete Methode aus diesem abgetrennt werden. Möglichkeiten zur Abtrennung des Katalysators sind zum Beispiel das Auswaschen mit einem nicht mit dem Produkt mischbaren Lösungsmittel, in welchem sich der Katalysator durch geeignete Wahl der Liganden besser löst als im Produkt. Gegebenenfalls wird der Katalysator durch mehrstufige Extraktion aus dem Produkt abgereichert. Als Extraktionsmittel wird bevorzugt ein auch für die Zielreaktion geeignetes Lösungsmittel wie Toluol, Benzol, Xylole, Alkane, wie Hexane, Heptane und Oktane, und azyklische oder zyklische Ether, wie Diethylether und Tetrahydrofuran eingesetzt, welches nach dem Einengen zusammen mit dem extrahierten Katalysator wieder für die Umsetzung eingesetzt werden kann. Möglich ist auch die Entfernung des Katalysators mit einem geeigneten Absorbermaterial. Eine Abtrennung kann auch durch Zufügen von Wasser zu der Produktphase erfolgen, falls die Reaktion in einem mit Wasser nicht mischbaren Lösungsmittel durchgeführt wird. Löst sich der Katalysator dabei bevorzugt in dem Lösungsmittel, kann er mit dem Lösungsmittel von der wässrigen Produktphase abgetrennt und gegebenenfalls erneut eingesetzt werden. Dies kann durch Wahl geeigneter Liganden bewirkt werden. Die resultierenden wässrigen Di-, Tri-, oder Polyamine können direkt als technische Aminlösungen eingesetzt werden. Es ist auch möglich, das Aminierungsprodukt durch Destillation vom Katalysator zu trennen.

Wird die Reaktion in einem Lösungsmittel durchgeführt, so kann dieses mit dem Aminierungsprodukt mischbar sein und nach der Reaktion durch Destillation abgetrennt werden. Es können auch Lösungsmittel eingesetzt werden, welche eine Mischungslücke mit den Aminierungsprodukten oder den Edukten aufweisen. Als geeignete Lösungsmittel hierfür seien beispielsweise Toluol, Benzol, Xylole, Alkane, wie Hexane, Heptane und Oktane, und azyklische oder zyklische Ether, wie Diethylether, Tetrahydrofuran und Dioxan genannt. Durch geeignete Wahl der Phosphinliganden löst sich der Katalysator bevorzugt in der Lösungsmittelphase, d.h. in der nicht-Produkt-enthaltenden Phase. Die Phosphinliganden können auch so gewählt werden, dass sich der Katalysator im Aminierungsprodukt löst. In diesem Fall lässt sich das Aminierungsprodukt durch Destillation vom Katalysator trennen.

Als Lösungsmittel kann auch das Produkt verwendet werden. Das Lösungsmittel kann auch unter den Reaktionsbedingungen mit den Edukten und dem Produkt mischbar sein und erst nach dem Abkühlen eine zweite flüssige Phase ausbilden, welche den Großteil des Katalysators enthält. Als Lösungsmittel, die diese Eigenschaft zeigen, seien beispielsweise Toluol, Benzol, Xylole, Alkane, wie Hexane, Heptane und Oktane, genannt. Der Katalysator kann dann zusammen mit dem Lösungsmittel abgetrennt und wieder verwendet werden. Die Produktphase kann auch in dieser Variante mit Wasser versetzt werden. Der in dem Produkt enthaltene Teil des Katalysators kann anschließend durch geeignete Absorbermaterialien wie beispielsweise Polyacrylsäure und deren Salze, sulfonierte Polystyrole und deren Salze, Aktivkohlen, Montmorillonite, Bentonite sowie Zeolithe abgetrennt werden oder aber in dem Produkt belassen werden.

Die Aminierungsreaktion kann auch zweiphasig durchgeführt werden. Bei der Ausführungsform der zweiphasigen Reaktionsführung eignen sich als unpolare Lösungsmittel besonders Toluol, Benzol, Xylole, Alkane, wie Hexane, Heptane und Oktane, in Kombination mit lipophilen Phosphinliganden am Übergangsmetallkatalysator, wodurch sich der Übergangsmetallkatalysator in der unpolaren Phase anreichert. Bei dieser Ausführungsform, in welcher das Produkt sowie das Reaktionswasser und ggf. nicht umgesetzte Edukte eine mit diesen Verbindungen angereicherte Zweitphase bilden, kann der Großteil des Katalysators durch einfache Phasentrennung von der Produktphase abgetrennt und wiederverwendet werden.

Falls flüchtige Nebenprodukte oder nicht umgesetzte Edukte oder auch das bei der Reaktion entstandene oder nach der Reaktion zur besseren Extraktion zugesetzte Wasser unerwünscht sind, können diese problemlos von dem Produkt durch Destillation abgetrennt werden.

Es kann auch vorteilhaft sein, das bei der Reaktion gebildete Wasser kontinuierlich aus dem Reaktionsgemisch zu entfernen. Das Reaktionswasser kann direkt durch Destillation aus dem Reaktionsgemisch oder als Azeotrop unter Zusatz eines geeigneten Lösungsmittels (Schleppers) und unter Verwendung eines Wasserabscheiders abgetrennt, oder durch Zusatz von Wasser entziehenden Hilfsstoffen entfernt werden.

Der Zusatz von Basen kann einen positiven Effekt auf die Produktbildung haben. Als geeignete Basen seien hier Alkalihydroxide, Erdalkalihydroxide, Alkalialkoholate, Erdalkalialkoholate, Alkali-Carbonate und Erdalkalicarbonate genannt, von welchen 0,01 bis 100 molare Äquivalente in Bezug auf den verwendeten Metallkatalysator eingesetzt werden können.

Ein weiterer Gegenstand der vorliegenden Erfindung ist die Verwendung eines Komplexkatalysators der allgemeinen Formel (I): 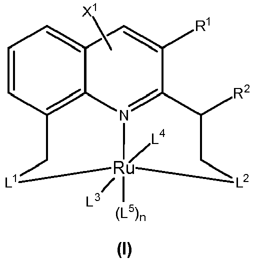 in der X¹, R¹, R², L¹, L², L³, L⁴, L⁵ und n die vorstehend beschriebenen Bedeutungen haben, wobei die vorstehend beschriebenen Bevorzugungen entsprechend gelten, als homogener Komplexkatalysator zur Herstellung von primären Amiden durch Alkoholaminierung von Alkoholen mit Ammoniak unter Wasserabspaltung.

Die Erfindung wird durch die nachfolgenden Beispiele verdeutlicht, ohne sie darauf zu beschränken.

### Beispiele

### Herstellung der Katalysatoren

### Herstellung des Komplexkatalysators in einem vorgelagerten Schritt (erfindungsgemäß; Kat1)

In einem 20mL Stahlautoklaven werden unter Argon-Atmosphäre [Ru(COD)(2-methylallyl)₂] (700mg, 2.19 mmol) und 4,5-Bis-[(dicyclohexylphosphanyl)-methyl]-acridin (1.48g, 2.47 mmol) in abolutem Toluol (10 ml) vorgelegt. Nachfolgend werden 40 bar Sythesegas (Volumenverhältnis CO/H₂=1:1) bei Raumtemperatur (20°C) aufgepresst. Anschließend wird auf 100°C erwärmt und für 20 Stunden bei dieser Temperatur gerührt. Der Stahlautoklav wird abgekühlt und entspannt und der Austrag, eine rote, klare Lösung, wird im Vakuum eingeengt. Der so erhaltene rote Rückstand wird aus THF gefällt. Durch Filtration werden 850mg eines roten Feststoffes isoliert. Der so erhaltene Komplexkatalysator wird nachfolgend in der Alkoholaminierung eingesetzt. Der Komplexkatalysator wurde wie folgt charakterisiert.
Kat.1: ¹H NMR (500 MHz, Benzol-d6): δ [ppm] = 8,4 (s, 2H, Ar-H), 8,2 (s, 1H, H9), 7,6-7,4 (4H, Ar-H), 4,7 (bs, 4H, -CH*H*-PCy₂), 3,6 (2H, -P(Cₐ*H*(CH₂)₅)₂), 2,5-1,0 (42H, Cy-H, -C*H*H-PCy₂); keine Hydrid-Signale im Bereich von -1 bis -30ppm; ³¹P{¹H} NMR (145,98 MHz, Benzol-d6): δ [ppm] = 70,6 (s, -CH₂-*P*(Cy)₂); ¹³C NMR (125 MHz, Benzol-d6): δ [ppm] = 210.7 (bs, CO), 147.9 (s, Ar-C), 137.7 (d, Ar-C), 134.3 (s, Ar-C), 133.2 (d, Ar-C), 125.5 (d, Ar-C), 126.8, 127.3, (2 Ar-C), 39.1 (d, CH), 30.8, 29.1, 27.6, 26.7 (4 t, 4 CH₂), IR (KBr): □_{CO} 1965, 1879, 1857 cm⁻¹.

### Herstellung des Komplexkatalysators in dem Reaktor zur Alkoholaminierung (erfindungsgemäß; Kat2)

In einem Reaktor zur Alkoholaminierung (160 ml Parr-Autoklav aus Edelstahl V4A) werden unter Argon-Atmosphäre [Ru₃(CO)₁₂], 4,5-Bis-[(dicyclohexylphosphanyl)-methyl]-acridin (der Donorligand wird in äquimolaren Mengen bezogen auf das in der ruthemiumhaltigen Katalysatorvorstufe enthaltene Ruthenium eingesetzt; die Mengen ergeben sich aus Tabelle 1) zusammen mit dem Lösungsmittel (tert-Amylalkohol), dem zu aminierenden Alkohol (Edukt) und Ammoniak vorgelegt. Anschließend wird die Alkoholaminierung gemäß der nachstehenden Vorschrift durchgeführt. Der Komplexkatalysator bildet sich in situ unter den Reaktionsbedingungen der Alkoholaminierung.

### Komplexkatalysator aus dem Stand der Technik (Vergleichsbeispiel; Kat3)

Als Komplexkatalysator (Kat3) wurde eine Verbindung der folgenden Formel eingesetzt. 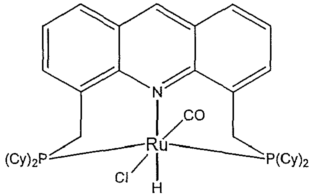 Dieser Komplexkatalysator ist in der PCT/EP2012/053584 beschrieben; Cy bedeutet Cyclohexyl).

### Homogen-katalysierte Alkoholaminierung von Alkoholen mit Ammoniak

Der Komplexkatalysator (Kat1, Kat2 oder Kat3) wird unter Argon-Atmosphäre zusammen mit dem Lösungsmittel und der umzusetzenden Alkohol in einem 160 ml Parr-Autoklaven (Edelstahl V4A) mit magnetisch gekoppeltem Schrägblattrührer (Rührgeschwindigkeit: 200-500 Umdrehungen/Minute) vorgelegt, beziehungsweise im Fall von Kat2 in situ hergestellt. Die angegebene Menge an Ammoniak wurde bei Raumtemperatur entweder vorkondensiert oder direkt aus der NH₃-Druckgasflasche zudosiert. Der Stahlautoklav wurde auf die angegebene Temperatur elektrisch aufgeheizt und für die angegebene Zeit unter Rühren (500 Umdrehungen/Minute) erhitzt (Innentemperaturmessung). Nach dem Abkühlen auf Raumtemperatur, Entspannung des Autoklaven und Ausgasen des Ammoniaks bei Normaldruck wurde die Reaktionsmischung mittels GC (30m DB1701 0,32mm 1,5µm) analysiert. Die Ergebnisse für die Aminierung von Isosorbid und Isomannid sind im Folgenden angegeben.

In Tabelle 1 sind die Ergebnisse der Umsetzung von Isosorbid und Isomannid zum Monoaminierungsprodukt (a) und zum Diaminierungsprodukt (b) gemäß der nachfolgenden Reaktionsgleichung gezeigt.

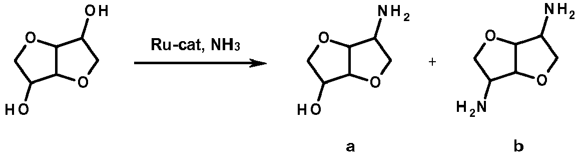

| Nr.^{a)} | Alkohol | Temp. [°C] | NH₃ [eq]^{d)} | Konzentration (mol/l) | Reaktionsdruck (bar) | Ru-System mol%^{e)} | Ru-Komplex | Umsatz Edukt^{b)} [%] | Selektivität ^{c)} | |
|---|---|---|---|---|---|---|---|---|---|---|
| | | | | | | | | | a [%] | b [%] |
| 1 | Isosorbid | 200 | 18 | 1 | 85,0 | 0,60 | Kat 2 | 99,9 | 3,8 | 93,4 |
| 2 | Isosorbid | 200 | 20 | 1 | 88,0 | 0,90 | Kat 2 | 99,3 | 0,3 | 96,5 |
| | | | | | | | | | | |
| 5 | Isosorbid | 180 | 18 | 2 | 83,6 | 0,90 | Kat 2 | 99,4 | 16,9 | 81,5 |
| 3 | Isosorbid | 180 | 18 | 1 | 72,0 | 1,20 | Kat 2 | 99,3 | 2,5 | 96,1 |
| 6 | Isosorbid | 180 | 32 | 1 | 104,0 | 0,90 | Kat 2 | 93,9 | 7,1 | 89,6 |
| 4 | Isosorbid | 180 | 6 | 2 | 49,1 | 0,90 | Kat 2 | 99,7 | 8,3 | 91,0 |
| 7 | Isosorbid | 180 | 6 | 4 | 64,6 | 0,90 | Kat 2 | 99,6 | 9,9 | 89,5 |
| 8 | Isosorbid | 180 | 6 | 6 | 76,4 | 0,90 | Kat 2 | 99,7 | 5,9 | 93,5 |
| | | | | | | | | | | |
| 9 | Isosorbid | 180 | 6 | 1 | 46,6 | 0,60 | Kat 3 | 98,05 | 82,76 | 14,09 |
| 10 | Isosorbid | 180 | 6 | 1 | 46,7 | 0,60 | Kat 2 | 96,86 | 32,08 | 66,54 |
| 11 | Isomannid | 180 | 6 | 1 | 44,8 | 0,30 | Kat 2 | 99,5 | 13,1 | 85,6 |
| 12 | Isosorbid | 180 | 6 | 2 | 53,7 | 0,30 | Kat 3 | 98,4 | 94,0 | 3,1 |
| 13 | Isosorbid | 180 | 6 | 1 | 46,0 | 0,30 | Kat 2 | 92,4 | 59,4 | 40,4 |
| 14 | Isosorbid | 180 | 6 | 1 | 46,3 | 0,30 | Kat 1 | 93,2 | 56,6 | 43,4 |
| a) Bedingungen, wenn nicht anders angegeben: 50 ml tert-Amylalkohol, Ansatzgröße 25 mmol Isosorbid oder Isomannid, Reaktionsdauer 20 h, b) Auswertung per GC (FI%), c) Produktselektivität bestimmt per GC, d) Molare Äquivalente NH₃ pro OH-Funktion am Substrat, e) mol% Ru-Katalysator pro OH-Funktion am Substrat | | | | | | | | | | |

## Patentansprüche

1. Verfahren zur Herstellung von primären Aminen durch Alkoholaminierung von Alkoholen mit Ammoniak unter Wasserabspaltung, wobei die Alkoholaminierung homogen-katalysiert in Gegenwart mindestens eines Komplexkatalysators durchgeführt wird, der Ruthenium sowie mindestens einen mindestens bidentalen Donorliganden, jedoch keinen anionischen Liganden enthält,
wobei der Komplexkatalysator ein Katalysator der allgemeinen Formel (I) ist: 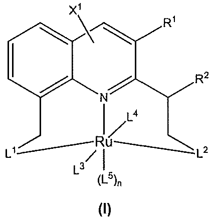 in der
L¹ und L² sind unabhängig voneinander PR^{a}R^{b} oder NR^{a}R^{b};
L³, L⁴ und L⁵ sind unabhängig voneinander einzähnige Zweielektronendonoren ausgewählt sind aus der Gruppe CO und PR^{a}R^{b}R^{c};
n ist eine ganze Zahl 0 oder 1;
R¹ und R² sind beide Wasserstoffe oder bilden gemeinsam mit den Kohlenstoffatomen, an die sie gebunden sind, einen Phenylring, der zusammen mit der Chinolinyleinheit der Formel I eine Acridinyleinheit bildet;
R, R^{a}, R^{b} und R^{c} sind unabhängig voneinander unsubstituiertes oder zumindest monosubstituiertes C₁-C₁₀-Alkyl, C₅-C₁₀-Cycloalkyl, C₅-C₁₀-Heterocyclyl, C₅-C₁₀-Aryl oder C₅-C₁₀-Heteroaryl,
wobei die Substituenten ausgewählt sind aus der Gruppe bestehend aus F, Cl, Br, OH, CN, NH₂ und C₁-C₁₀-Alkyl;
X¹ stellt ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit dar,
wobei X¹ unabhängig voneinander ausgewählt ist aus der Gruppe bestehend aus Wasserstoff, F, Cl, Br, I, OH, NH₂, NO₂, NC(O)R, C(O)NR₂, OC(O)R, C(O)OR, CN und unsubstituiertem oder zumindest monosubstituiertem C₁-C₁₀-Alkoxy, C₁-C₁₀-Alkyl, C₅-C₁₀-Cycloalkyl, C₅-C₁₀-Heterocyclyl, C₅-C₁₀-Aryl und C₅-C₁₀-Heteroaryl,
wobei die Substituenten ausgewählt sind aus der Gruppe bestehend aus F, Cl, Br, OH, CN, NH₂ und C₁-C₁₀-Alkyl.

2. Verfahren nach Anspruch 1, wobei R¹ und R² beide Wasserstoff sind und der Komplexkatalysator ein Katalysator der allgemeinen Formel (IV) ist: 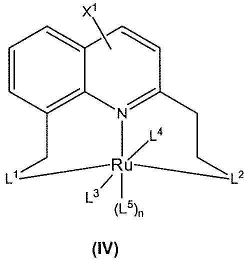 in der X¹, L¹, L², L³, L⁴, L⁵ und n die in Anspruch 2 genannten Bedeutungen aufweisen.

3. Verfahren nach Anspruch 1, wobei R¹ und R² gemeinsam mit den Kohlenstoffatomen, an die sie gebunden sind, ein Phenylring bilden, der zusammen mit der Chinolinyleinheit der Formel I eine Acridinyleinheit bildet und der Komplexkatalysator ein Katalysator der Formel (V) ist: 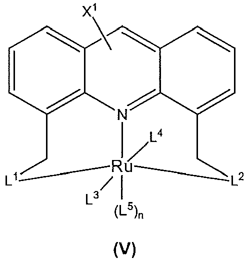 in der X¹, L¹, L², L³, L⁴, L⁵ und n die in Anspruch 2 genannten Bedeutungen aufweisen.

4. Verfahren nach Anspruch 1, wobei der Komplexkatalysator aus der Gruppe bestehend aus Katalysatoren der Formel (VI), (VII), (VIII), (IX), (X), (XI), (XII) und (XIII) ausgewählt ist: 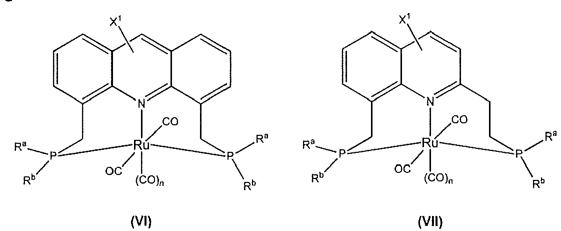 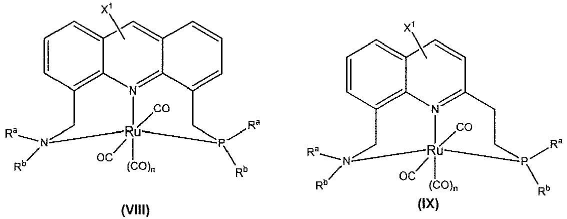 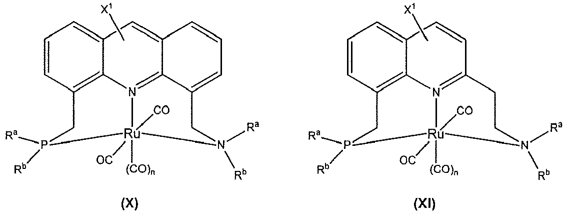 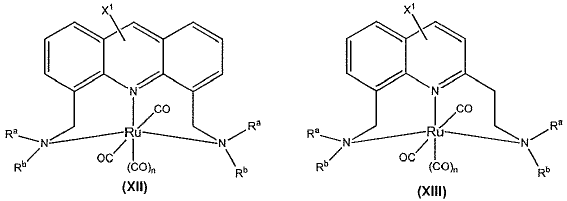 in denen X¹, R^{a}, R^{b} und n die in Anspruch 1 genannten Bedeutungen aufweisen.

5. Verfahren nach einem der Ansprüche 1 bis 4, wobei X¹ und n die in Anspruch 2 genannten Bedeutungen aufweisen und R^{a} und R^{b} unsubstituiertes Cyclohexyl sind.

6. Verfahren nach einem der Ansprüche 1 oder 3, wobei der Komplexkatalysator ein Katalysator der Formel (XIVb) ist: 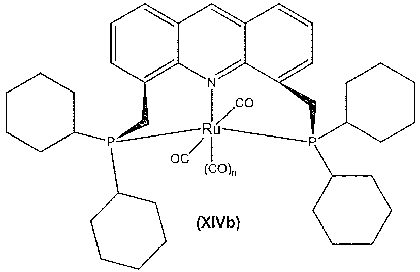 in der n eine ganze Zahl 0 oder 1 ist.

7. Verfahren nach einem der Ansprüche 1 bis 6, wobei im Komplexkatalysator n = 0 ist.

8. Verfahren nach einem der Ansprüche 1 bis 7, wobei die Alkohole mindestens eine primäre oder mindestens eine sekundäre Alkylgruppe aufweisen.

9. Verfahren nach einem der Ansprüche 1 bis 8, wobei es sich bei den primären Aminen um Monoamine, Alkanolamine, Diamine, Triamine oder Polyamine handelt.

10. Verfahren nach einem der Ansprüche 1 bis 9, wobei die Alkohole mindestens zwei sekundäre Alkoholgruppen aufweisen.

11. Verfahren nach einem der Ansprüche 1 bis 10, wobei die Alkohole ausgewählt sind aus der Gruppe bestehend aus Isosorbid, Isomannid, Isoidid und Mischungen dieser Verbindungen.

12. Verfahren nach einem der Ansprüche 1 bis 11, wobei der Komplexkatalysator (I) in dem Reaktor aus mindestens einer rutheniumhaltigen Katalysatorvorstufe und mindestens einem Donorliganden der allgemeinen Formel (la) 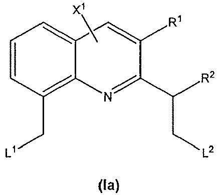 in der X¹, L¹, L², R¹ und R² die in Anspruch 1 genannten Bedeutungen aufweisen, in Gegenwart eines Lösungsmittels hergestellt wird.

13. Verfahren gemäß Anspruch 12, wobei die rutheniumhaltige Katalysatorvorstufe ausgewählt ist aus der Gruppe bestehend aus [Ru₃(CO)₁₂], [Ru(CO)₄(ethylen)], [Ru(COD)(OAC)₂], [Ru(COD)(2-methylallyl)₂], [Ru(CO)₃Cl₂]₂, [RuCl₃*H₂O], [Ru(acetylacetonat)_{3]}, [Ru(PPh₃)₃(CO)(H)Cl], [Ru(PPh₃)₃(CO)Cl₂], [Ru(PPh₃)₃(CO)(H)₂], [Ru(PPh₃)₃Cl₂] und [Ru(COD)Cl₂]₂.

14. Verfahren zur Herstellung eines Komplexkatalysators (I) durch Umsetzen mindestens einer rutheniumhaltigen Katalysatorvorstufe ausgewählt aus der Gruppe bestehend aus [Ru₃(CO)₁₂], [Ru(CO)₄(ethylen)], [Ru(COD)(OAc)₂], [Ru(COD)(2-methylallyl)₂], [Ru(CO)₃Cl₂]₂, [RuCl₃*H₂O], [Ru(acetylacetonat)_{3]}, [Ru(PPh₃)₃(CO)(H)Cl], [Ru(PPh₃)₃(CO)Cl₂], [Ru(PPh₃)₃(CO)(H)₂], [Ru(PPh₃)₃Cl₂] und [Ru(COD)Cl₂]₂ mit mindestens einem Donorliganden der allgemeinen Formel (la) in Gegenwart eines Lösungsmittels sowie gegebenenfalls in Gegenwart von Kohlenmonoxid oder Synthesegas bei einem Druck im Bereich von 20 bis 60 bar, wobei der Komplexkatalysator (I) und der mindestens eine Donorligand der allgemeinen Formel (la) die in den Ansprüchen 1 und 12 genannten Bedeutungen aufweisen.

## Claims

1. A process for preparing primary amines by alcohol amination of alcohols with ammonia with the elimination of water, where the alcohol amination is carried out under homogeneous catalysis in the presence of at least one complex catalyst which comprises ruthenium and at least one at least bidental donor ligand, but no anionic ligands,
where the complex catalyst is a catalyst of the general formula (I): 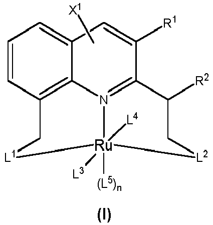 in which
L¹ and L² independently of one another are PR^{a}R^{b} or NR^{a}R^{b};
L³, L⁴ and L⁵ independently of one another are monodentate two-electron donors selected from the group CO and PR^{a}R^{b}R^{c};
n is an integer 0 or 1;
R¹ and R² are both hydrogens or, together with the carbon atoms to which they are bonded, form a phenyl ring which, together with the quinolinyl unit of the formula I, forms an acridinyl unit;
R, R^{a}, R^{b} and R^{c} independently of one another are unsubstituted or at least monosubstituted C₁-C₁₀-alkyl, C₅-C₁₀-cycloalkyl, C₅-C₁₀-heterocyclyl, C₅-C₁₀-aryl or C₅-C₁₀-heteroaryl,
where the substituents are selected from the group consisting of F, Cl, Br, OH, CN, NH₂ and C₁-C₁₀-alkyl;
X¹ is one, two, three, four, five, six or seven substituents on one or more atoms of the acridinyl unit or one, two, three, four or five substituents on one or more atoms of the quinolinyl unit,
where X¹, independently of the others, is selected from the group consisting of hydrogen, F, Cl, Br, I, OH, NH₂, NO₂, NC(O)R, C(O)NR₂, OC(O)R, C(O)OR, CN and unsubstituted or at least monosubstituted C₁-C₁₀-alkoxy, C₁-C₁₀-alkyl, C₅-C₁₀-cycloalkyl, C₅-C₁₀-heterocyclyl, C₅-C₁₀-aryl and C₅-C₁₀-heteroaryl,
where the substituents are selected from the group consisting of F, Cl, Br, OH, CN, NH₂ and C₁-C₁₀-alkyl.

2. The process according to claim 1, where R¹ and R² are both hydrogen and the complex catalyst is a catalyst of the general formula (IV): 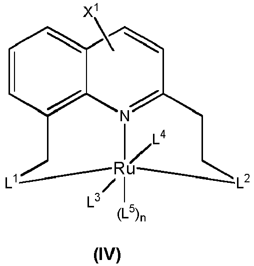 in which X¹, L¹, L², L³, L⁴, L⁵ and n have the meanings given in claim 2.

3. The process according to claim 1, where R¹ and R², together with the carbon atoms to which they are bonded, form a phenyl ring which, together with the quinolinyl unit of the formula I, forms an acridinyl unit, and the complex catalyst is a catalyst of the formula (V): 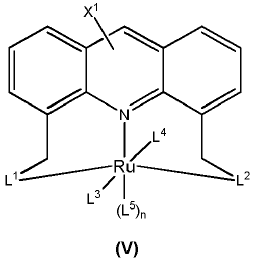 in which X¹, L¹, L², L³, L⁴, L⁵ and n have the meanings given in claim 2.

4. The process according to claim 1, where the complex catalyst is selected from the group consisting of catalysts of the formulae (VI), (VII), (VIII), (IX), (X), (XI), (XII) and (XIII): 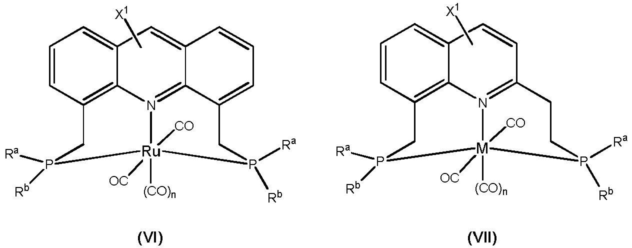 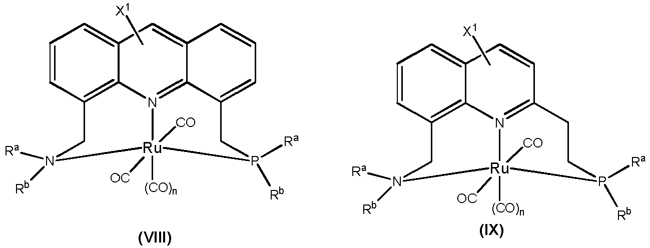 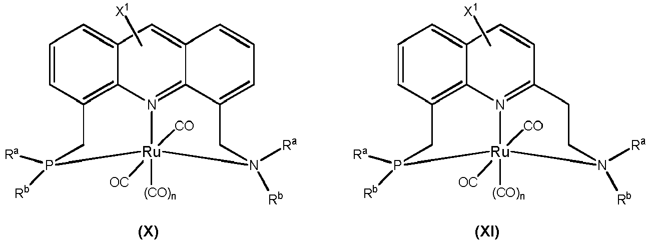 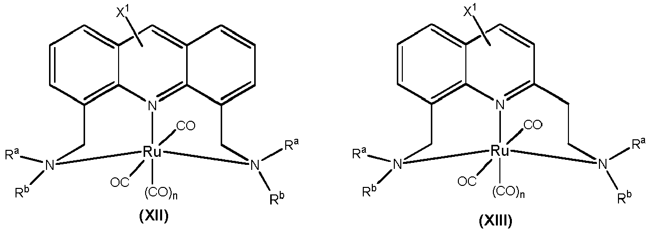 in which X¹, R^{a}, R^{b} and n have the meanings given in claim 1.

5. The process according to any one of claims 1 to 4, where X¹ and n have the meanings given in claim 2 and R^{a} and R^{b} are unsubstituted cyclohexyl.

6. The process according to any one of claims 1 or 3, where the complex catalyst is a catalyst of the formula (XIVb) : 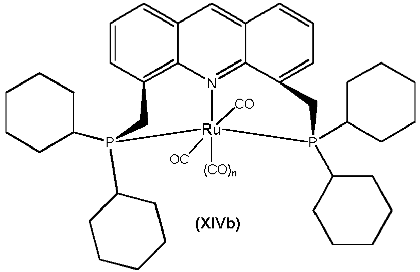 in which n is an integer 0 or 1.

7. The process according to any one of claims 1 to 6, where n = 0 in the complex catalyst.

8. The process according to any one of claims 1 to 7, where the alcohols have at least one primary or at least one secondary alkyl group.

9. The process according to any one of claims 1 to 8, where the primary amines are monoamines, alkanolamines, diamines, triamines or polyamines.

10. The process according to any one of claims 1 to 9, where the alcohols have at least two secondary alcohol groups.

11. The process according to any one of claims 1 to 10, where the alcohols are selected from the group consisting of isosorbide, isomannide, isoidide and mixtures of these compounds.

12. The process according to any one of claims 1 to 11, where the complex catalyst (I) is prepared in the reactor from at least one ruthenium-containing catalyst precursor and at least one donor ligand of the general formula (Ia) 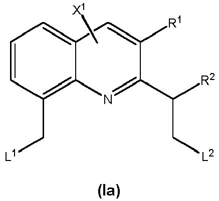 in which X¹, L¹, L², R¹ and R² have the meanings given in claim 1, in the presence of a solvent.

13. The process according to claim 12 where the ruthenium-containing catalyst precursor is selected from the group consisting of [Ru₃(CO)₁₂], [Ru(CO)₄(ethylene)], [Ru(COD)(OAc)₂], [Ru(COD)(2-methylallyl)₂], [Ru(CO)₃Cl₂]₂, [RuCl₃*H₂O], [Ru(acetylacetonate)₃], [Ru(PPh₃)₃(CO)(H)Cl], [Ru(PPh₃)₃(CO)Cl₂], [Ru(PPh₃)₃(CO)(H)₂], [Ru(PPh₃)₃Cl₂] and [Ru(COD)Cl₂]₂.

14. A process for preparing a complex catalyst (I) by reacting at least one ruthenium-containing catalyst precursor selected from the group consisting of [Ru₃(CO)₁₂], [Ru(CO)₄(ethylene)], [Ru(COD)(OAc)₂], [Ru(COD)(2-methylallyl)₂], [Ru(CO)₃Cl₂]₂, [RuCl₃*H₂O], [Ru(acetylacetonate)₃], [Ru(PPh₃)₃(CO)(H)Cl], [Ru(PPh₃)₃(CO)Cl₂], [Ru(PPh₃)₃(CO)(H)₂], [Ru(PPh₃)₃Cl₂] and [Ru(COD)Cl₂]₂ with at least one donor ligand of the general formula (Ia) in the presence of a solvent and optionally in the presence of carbon monoxide or synthesis gas at a pressure in the range from 20 to 60 bar, where the complex catalyst (I) and the at least one donor ligand of the general formula (Ia) have the meanings given in claims 1 and 12.

## Revendications

1. Procédé de fabrication d'amines primaires par amination d'alcools avec de l'ammoniac avec clivage d'eau, l'amination d'alcools étant réalisée sous catalyse homogène en présence d'au moins un catalyseur complexe, qui contient du ruthénium et au moins un ligand donneur au moins bidentate, mais toutefois pas de ligand anionique,
le catalyseur complexe étant un catalyseur de formule générale (I) : 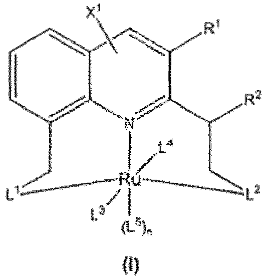 dans laquelle
L¹ et L² représentent indépendamment l'un de l'autre PR^{a}R^{b} ou NR^{a}R^{b} ;
L³, L⁴ et L⁵ représentent indépendamment les uns des autres des donneurs de deux électrons monodentates choisis dans le groupe constitué par CO et PR^{a}R^{b}R^{c} ;
n est un nombre entier 0 ou 1 ;
R¹ et R² représentent tous les deux l'hydrogène ou forment ensemble avec les atomes de carbone auxquels ils sont reliés un cycle phényle, qui forme conjointement avec l'unité quinolinyle de la formule I une unité acridinyle ;
R, R^{a}, R^{b} et R^{c} représentent indépendamment les uns des autres alkyle en C₁-C₁₀, cycloalkyle en C₅-C₁₀, hétérocyclyle en C₅-C₁₀, aryle en C₅-C₁₀ ou hétéroaryle en C₅-C₁₀ non substitués ou au moins monosubstitués,
les substituants étant choisis dans le groupe constitué par F, Cl, Br, OH, CN, NH₂ et alkyle en C₁-C₁₀ ;
X¹ représente un, deux, trois, quatre, cinq, six ou sept substituants sur un ou plusieurs atomes de l'unité acridinyle ou un, deux, trois, quatre ou cinq substituants sur un ou plusieurs atomes de l'unité quinolinyle,
les X¹ étant choisis indépendamment les uns des autres dans le groupe constitué par hydrogène, F, Cl, Br, I, OH, NH₂, NO₂, NC(O)R, C(O)NR₂, OC(O)R, C(O)OR, CN et alcoxy en C₁-C₁₀, alkyle en C₁-C₁₀, cycloalkyle en C₅-C₁₀, hétérocyclyle en C₅-C₁₀, aryle en C₅-C₁₀ et hétéroaryle en C₅-C₁₀ non substitués ou au moins monosubstitués,
les substituants étant choisis dans le groupe constitué par F, Cl, Br, OH, CN, NH₂ et alkyle en C₁-C₁₀.

2. Procédé selon la revendication 1, dans lequel R¹ et R² représentent tous les deux l'hydrogène et le catalyseur complexe est un catalyseur de formule générale (IV) : 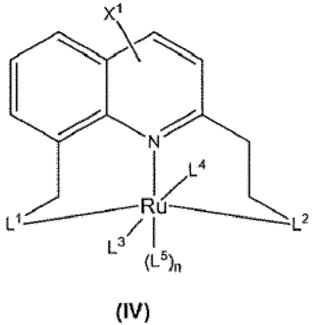 dans laquelle X¹, L¹, L², L³, L⁴, L⁵ et n ont les significations indiquées dans la revendication 2.

3. Procédé selon la revendication 1, dans lequel R¹ et R² forment ensemble avec les atomes de carbone auxquels ils sont reliés un cycle phényle, qui forme conjointement avec l'unité quinolinyle de la formule I une unité acridinyle, et le catalyseur complexe est un catalyseur de formule (V) : 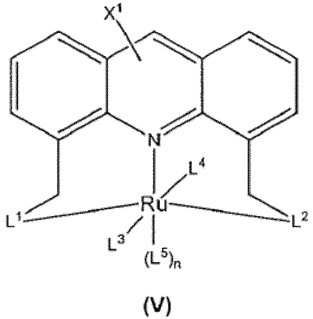 dans laquelle X¹, L¹, L², L³, L⁴, L⁵ et n ont les significations indiquées dans la revendication 2.

4. Procédé selon la revendication 1, dans lequel le catalyseur complexe est choisi dans le groupe constitué par les catalyseurs de formule (VI), (VII), (VIII), (IX), (X), (XI), (XII) et (XIII) : 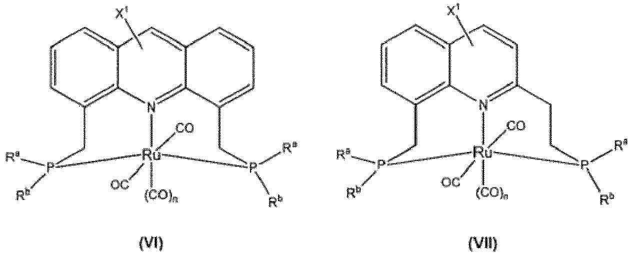 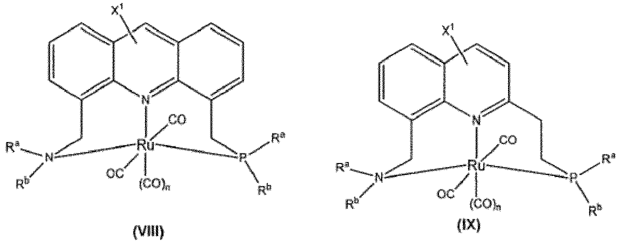 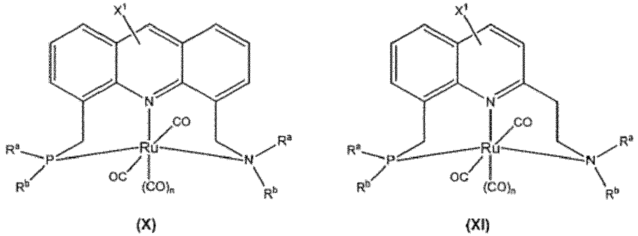 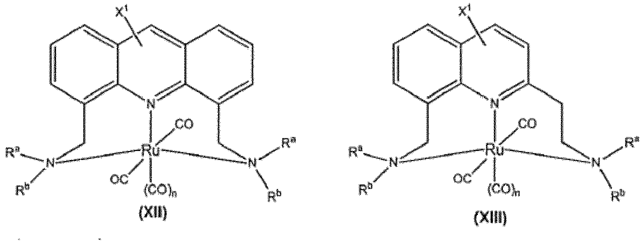 dans lesquelles X¹, R^{a}, R^{b} et n ont les significations indiquées dans la revendication 1.

5. Procédé selon l'une quelconque des revendications 1 à 4, dans lequel X¹ et n ont les significations indiquées dans la revendication 2, et R^{a} et R^{b} représentent un cyclohexyle non substitué.

6. Procédé selon l'une quelconque des revendications 1 ou 3, dans lequel le catalyseur complexe est un catalyseur de formule (XIVb) : 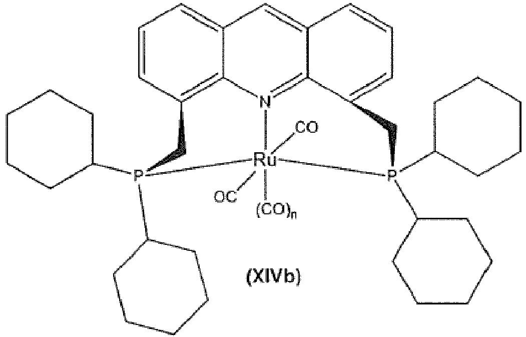 dans laquelle n est un nombre entier 0 ou 1.

7. Procédé selon l'une quelconque des revendications 1 à 6, dans lequel, dans le catalyseur complexe, n = 0.

8. Procédé selon l'une quelconque des revendications 1 à 7, dans lequel les alcools comprennent au moins un groupe alkyle primaire ou au moins un groupe alkyle secondaire.

9. Procédé selon l'une quelconque des revendications 1 à 8, dans lequel les amines primaires sont des monoamines, des alcanolamines, des diamines, des triamines ou des polyamines.

10. Procédé selon l'une quelconque des revendications 1 à 9, dans lequel les alcools comprennent au moins deux groupes alcool secondaires.

11. Procédé selon l'une quelconque des revendications 1 à 10, dans lequel les alcools sont choisis dans le groupe constitué par l'isosorbide, l'isomannide, l'isoidide et les mélanges de ces composés.

12. Procédé selon l'une quelconque des revendications 1 à 11, dans lequel le catalyseur complexe (I) est fabriqué dans le réacteur à partir d'au moins un précurseur de catalyseur contenant du ruthénium et d'au moins un ligand donneur de formule générale (Ia) 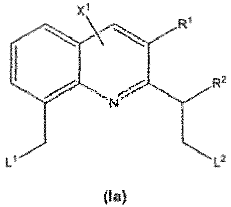 dans laquelle X¹, L¹, L², R¹ et R² ont les significations indiquées dans la revendication 1, en présence d'un solvant.

13. Procédé selon la revendication 12, dans lequel le précurseur de catalyseur contenant du ruthénium est choisi dans le groupe constitué par [Ru₃(CO)₁₂], [Ru(CO)₄(éthylène)], [Ru(COD)(OAc)₂], [Ru(COD)(2-méthylallyl)₂], [Ru(CO)₃Cl₂]₂, [RuCl₃*H₂O], [Ru(acétylacétonate)₃], [Ru(PPh₃)₃(CO)(H)Cl], [Ru(PPh₃)₃(CO)Cl₂], [Ru(PPh₃)₃(CO)(H)₂], [Ru(PPh₃)₃Cl₂] et [Ru(COD)Cl₂]₂.

14. Procédé de fabrication d'un catalyseur complexe (I) par mise en réaction d'au moins un précurseur de catalyseur contenant du ruthénium choisi dans le groupe constitué par [Ru₃(CO)₁₂], [Ru(CO)₄(éthylène)], [Ru(COD)(OAc)₂], [Ru(COD)(2-méthylallyl)₂], [Ru(CO)₃Cl₂]₂, [RuCl₃*H₂O], [Ru(acétylacétonate)₃], [Ru(PPh₃)₃(CO)(H)Cl], [Ru(PPh₃)₃(CO)Cl₂], [Ru(PPh₃)₃(CO)(H)₂], [Ru(PPh₃)₃Cl₂] et [Ru(COD)Cl₂]₂ avec au moins un ligand donneur de formule générale (Ia) en présence d'un solvant et éventuellement en présence de monoxyde de carbone ou d'un gaz de synthèse à une pression dans la plage allant de 20 à 60 bar, le catalyseur complexe (I) et ledit au moins un ligand donneur de formule générale (Ia) ayant les significations indiquées dans les revendications 1 et 12.
